(19) Europäisches Patentamt
European Patent Office
Office européen des brevets

(11) EP 1 957 122 B1

(12) **EUROPEAN PATENT SPECIFICATION**

(45) Date of publication and mention of the grant of the patent:
**02.01.2013 Bulletin 2013/01**

(21) Application number: **06827240.0**

(22) Date of filing: **01.11.2006**

(51) Int Cl.:
***A61L 15/24*** *(2006.01)* ***A61L 15/60*** *(2006.01)*

(86) International application number:
**PCT/US2006/042596**

(87) International publication number:
**WO 2007/067277 (14.06.2007 Gazette 2007/24)**

(54) **ARTICLES COMPRISING FLEXIBLE SUPERABSORBENT BINDER POLYMER COMPOSITION**

ARTIKEL MIT EINER FLEXIBLEN SUPERSAUGFÄHIGEN BINDEMITTEL-POLYMER-ZUSAMMENSETZUNG

ARTICLES COMPRENANT UNE COMPOSITION POLYMERE LIANTE, SUPERABSORBANTE, FLEXIBLE

(84) Designated Contracting States:
**BE DE FR GB NL**

(30) Priority: **02.12.2005 US 292997**

(43) Date of publication of application:
**20.08.2008 Bulletin 2008/34**

(73) Proprietor: **KIMBERLY-CLARK WORLDWIDE, INC.**
**Neenah, WI 54956 (US)**

(72) Inventors:
- **SOERENS, Dave Allen**
**Neenah, WI 54956 (US)**
- **LANG, Angela Jones**
**High Point, NC 27265 (US)**
- **AHMED, Iqbal**
**Greensboro, NC 27410 (US)**
- **SMITH, Scott J.**
**Greensboro, NC 27407 (US)**

(74) Representative: **Beacham, Annabel Rose**
**Dehns**
**St Bride's House**
**10 Salisbury Square**
**London**
**EC4Y 8JD (GB)**

(56) References cited:
**US-A1- 2004 018 365**
**US-A1- 2004 019 166**
**US-A1- 2004 106 721**
**US-A1- 2004 116 014**

EP 1 957 122 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## BACKGROUND

Articles are an essential part of people's lives. For example, absorbent articles can be useful for absorbing many types of fluids, including fluids secreted or eliminated by the human body. Superabsorbent materials are frequently used in absorbent articles to help improve the absorbent properties of such articles. Superabsorbent materials are generally polymer based and are available in many forms, such as powders, granules, microparticles, films and fibers, for example. Upon contact with fluids, such superabsorbent materials swell by absorbing the fluids into their structures. In general, superabsorbent materials can quickly absorb fluids insulted into such articles, and can retain such fluids to prevent leakage and help provide a dry feel, even after fluid insult.

There is continuing effort to improve the performance of articles, such as absorbent articles. One desire is to make absorbent articles thinner. Another desire is to make absorbent articles more flexible. Still another desire is to increase the integrity of absorbent articles, in both a wet and dry condition. Yet another desire is to increase the absorbent intake and/or the absorbent capacity of such articles. One method for improving absorbent properties could be to increase the amount of superabsorbent material in the article. However, an increase in superabsorbent material content can increase the thickness of the article, increase the shakeout of material, and likewise can decrease the flexibility and/or the integrity of the article. Therefore, there is a need for an article, such as an absorbent article, which achieves desired absorbent properties while maintaining desired thinness and/or flexibility and/or integrity in both a wet and dry condition.

A known approach for making hydrophilic polymers more functional upon exposure to aqueous fluid is to crosslink the water-soluble polymers. As a result of crosslinking, the material becomes swellable, and no longer soluble, in aqueous fluid. However, crosslinked polymers are difficult to apply to substrates or to establish intimate contact with surfaces because the crosslinked polymers are solid materials and have little or no ability to flow. Some of the crosslinked materials are fairly stiff, and inhibit the flexibility of the absorbent product.

What is therefore needed is a hydrophilic polymer that has latent crosslinking capability and which can be produced at an attractive cost. Such polymers could be easily applied, like a water-soluble polymer, since the hydrophilic polymer would be capable of flow prior to crosslinking. Latent crosslinking capability would also provide a simple means of crosslinking the polymer after the polymer has established intimate contact with substrates or has formed a desired final shape or form. There is also a need or desire for such a polymer which has a suitable level of flexibility.

US2004/0018365 discloses absorbent structures comprising a flexible absorbent binder bound to a substrate layer. The binder is formed by crosslinking a composition including 15 to 99.9% by mass monoethylenically unsaturated units, 0.1 to 20% by mass ester units selected from acrylate and methacrylate ester units that include an alkoxysilane functionality and zero to 75% by mass of units selected from polyolefin glycol and polyolefin oxide units.

US2004/0106721 discloses an absorbent binder desiccant composition comprising a cross-linkable water-soluble ionic polymer and at least one desiccant.

US2004/0019166 discloses a method of making an absorbent binder composition. The method comprises combining a first aqueous monomer solution containing a reducing polymerization initiator and a second aqueous monomer solution containing an oxidizing polymerizing initiator, and inducing cross-linking of the resultant binder composition by removal of water.

US2004/0116014 discloses an absorbent composite comprising a substrate and an absorbent adhesive composition, wherein at least a portion of the substrate is coated with the absorbent adhesive composition and the substrate is folded to provide a plurality of panels.

## SUMMARY

In response to the needs discussed above, an absorbent article according to claim 1, such as a sanitary napkin or a wound-care article is provided. More particularly, the article comprises a backsheet and optionally a topsheet, and an absorbent layer that includes a flexible superabsorbent binder polymer composition and optionally a substrate. The absorbent layer can be in contact with a planar surface of the backsheet and/or the optional topsheet. Alternatively, the flexible superabsorbent binder polymer composition may be positioned between both a backsheet and a topsheet. In other aspects, the absorbent layer may function as the backsheet or the topsheet.

The absorbent article of the present invention comprises a backsheet and an absorbent layer adjacent to and in facing relationship with the backsheet. The absorbent layer includes a flexible superabsorbent binder polymer composition formed by the reaction of a monomer solution including at least 15% by mass monoethylenically unsaturated monomer selected from carboxylic acid, carboxylic acid salts, sulphonic acid, sulphonic acid salts, phosphoric acid, or phosphoric acid salts; an acrylate or methacrylate ester that contains an alkoxysilane functionality; a plasticizer or a copolymerizable hydrophilic glycol containing an ester monomer; an initiator system; a chain transfer agent; a transition metal salt; and a neutralizing agent. The unsaturated monomer is neutralized to at least 25 mol%. The flexible superabsorbent binder polymer composition has a residual monoethylenically unsaturated monomer content of less than 1000 ppm. The flexible superabsorbent binder polymer composition has a weight average molecular weight of from 100,000 to 650,000 and a viscosity after 16 hours of less than 10,000 cps. In some aspects of this embodiment, the absorbent layer can further comprise a substrate adjacent to and in facing relationship with the flexible superabsorbent binder polymer composition. In other aspects of this embodiment, the absorbent article can include a topsheet, where the topsheet is positioned such that the absorbent layer is positioned between the topsheet and the backsheet.

The result is an article which exhibits improved performance as well as greater comfort and confidence among the user.

Numerous other features and advantages of the present invention will appear from the following description. In the description, reference is made to exemplary embodiments of the invention. Such embodiments do not represent the full scope of the invention. Reference should therefore be made to the claims herein for interpreting the full scope of the invention.

## FIGURES

The foregoing and other features, aspects and advantages of the present invention will become better understood with regard to the following description, appended claims and accompanying drawings where:

FIGS 1A and 1B are each a cross-section of an absorbent layer of the present invention having a substrate layer and an absorbent layer.

FIG 1C is a cross-section of an absorbent layer of the present invention having a substrate layer impregnated with an absorbent material.

FIGS 2A and 2B are each a cross-section of an absorbent layer of the present invention having a substrate layer impregnated with the absorbent material and an additional layer.

FIGS 2C and 2D are each a cross-section of an absorbent layer of the present invention having a substrate layer, an absorbent layer and an additional layer.

FIGS 3A and 3B are each a cross-section of an absorbent layer of the present invention having a substrate layer impregnated with the absorbent material and two additional layers.

FIG 4 is a representative, partially cut-away, top view of a body-facing side of an absorbent article which includes the flexible superabsorbent binder polymer composition of the present invention.

FIG 5A is a cross-section side view of an absorbent bandage of the present invention.

FIG 5B is a top perspective view of an absorbent bandage of the present invention.

FIG 6 is a top perspective view of an absorbent bed or furniture liner of the present invention.

Repeated use of reference characters in the present specification and drawings is intended to represent the same or analogous features or elements of the present invention.

## DEFINITIONS

It should be noted that, when employed in the present disclosure, the terms "comprises," "comprising" and other derivatives from the root term "comprise" are intended to be open-ended terms that specify the presence of any stated features, elements, integers, steps, or components, and are not intended to preclude the presence or addition of one or more other features, elements, integers, steps, components, or groups thereof.

The term "absorbent article" generally refers to a device which can absorb and contain fluids. For example, personal care absorbent articles refer to devices which are placed against or near the skin to absorb and contain the various fluids discharged from the body. The term "disposable" is used herein to describe absorbent articles that are not intended to be laundered or otherwise restored or reused as an absorbent article after a single use. Examples of such disposable absorbent articles include, but are not limited to, personal care absorbent articles, health/medical absorbent articles, and household/industrial absorbent articles.

The term "binder" includes materials that are capable of attaching themselves to a substrate or are capable of

attaching other substances to a substrate.

The term "coform" is intended to describe a blend of meltblown fibers and cellulose fibers that is formed by air forming a meltblown polymer material while simultaneously blowing air-suspended cellulose fibers into the stream of meltblown fibers. The coform material may also include other materials, such as superabsorbent materials. The meltblown fibers containing wood fibers are collected on a forming surface, such as provided by a foraminous belt. The forming surface may include a gas-pervious material, such as spunbonded fabric material, that has been placed onto the forming surface.

As used herein, the term "connected" is intended to mean that two or more members are directly or indirectly connected to one another. When two or more members are directly connected to one another, it is meant that the two members are in direct contact with one another, without an intervening member or structure. When two or more members are indirectly connected to one another, it is meant that the two members are not in direct contact with one another, and may have an intervening member or structure between the two or more members connected to one another.

The phrase "complex fluids" describes fluids generally characterized as being viscoelastic comprising multiple components having inhomogeneous physical and/or chemical properties. It is the inhomogeneous properties of the multiple components that challenge the efficacy of an absorbent or adsorbent material in the handling of complex liquids. In contrast with complex fluids, simple fluids, such as, for example, urine, physiological saline, water and the like, are generally characterized as being relatively low-viscosity and comprising one or more components having homogeneous physical and/or chemical properties. As a result of having homogeneous properties, the one or more components of simple fluids behave substantially similarly during absorption or adsorption, although some components of the simple fluids may be absorbed or adsorbed more readily than others. Although a complex liquid is generally characterized herein as including specific components having inhomogeneous properties, each specific component of a complex liquid generally has homogeneous properties. Consider for example a representative complex body-liquid having three specific components: red blood cells, blood protein molecules and water molecules. Upon examination, one skilled in the art could easily distinguish between each of the three specific components according to their generally inhomogeneous properties. Moreover, when examining a particular specific component such as the red blood cell component, one skilled in the art could easily recognize the generally homogeneous properties of the red blood cells.

The term "fluid" refers to a substance in the form of a liquid or gas at room temperature and atmospheric pressure.

The term "fluid impermeable," when used to describe a layer or laminate, means that fluid such as water or bodily fluids will not pass substantially through the layer or laminate under ordinary use conditions in a direction generally perpendicular to the plane of the layer or laminate at the point of fluid contact.

The term "health/medical articles" includes a variety of professional and consumer health-care products including, but not limited to, products for applying hot or cold therapy, medical gowns (i.e., protective and/or surgical gowns), surgical drapes, caps, gloves, face masks, bandages, wound dressings, wipes, covers, containers, filters, disposable garments and bed pads, medical absorbent garments, underpads, and the like.

The term "household/industrial articles" include construction and packaging supplies, products for cleaning and disinfecting, wipes, covers, filters, towels, disposable cutting sheets, bath tissue, facial tissue, nonwoven roll goods, home-comfort products including pillows, pads, mats, cushions, furniture liners and pads, masks and body care products such as products used to cleanse or treat the skin, laboratory coats, cover-alls, trash bags, stain removers, topical compositions, pet care absorbent liners, laundry soil/ink absorbers, detergent agglomerators, lipophilic fluid separators, and the like.

The terms "hydrophilic" and "wettable" are used interchangeably to refer to a material having a contact angle of water in air of less than 90 degrees. The term "hydrophobic" refers to a material having a contact angle of water in air of at least 90 degrees. For the purposes of this application, contact angle measurements are determined as set forth in Robert J. Good and Robert J. Stromberg, Ed., in "Surface and Colloid Science - Experimental Methods," Vol. II, (Plenum Press, 1979), herein incorporated by reference in a manner consistent with the present disclosure.

The term "insult target zone" refers to an area of an absorbent article or layer where it is particularly desirable for the majority of a fluid insult, such as urine, menses, or bowel movement, to initially contact. In particular, for an absorbent article or layer with one or more fluid insult points in use, the insult target zone refers to the area of the absorbent article or layer extending a distance equal to 15% of the total length of the composite from each insult point in both directions.

The term "knife over roll coating" refers to a process in which a knife is positioned, with a specified gap, above a substrate that is moving beneath the knife on a moving roll. In this manner, the knife spreads a specified thickness of coating material onto the substrate.

The term "layer" when used in the singular can have the dual meaning of a single element or a plurality of elements.

The term "materials" when used in the phrase "superabsorbent materials," refers generally to discrete units. The units can comprise particles, granules, fibers, flakes, agglomerates, rods, spheres, needles, particles coated with fibers or other additives, pulverized materials, powders, films, and the like, as well as combinations thereof. The materials

can have any desired shape such as, for example, cubic, rod-like, polyhedral, spherical or semi-spherical, rounded or semi-rounded, angular, irregular, etc. Additionally, superabsorbent materials may be composed of more than one type of material.

The term "meltblown fibers" refers to fibers formed by extruding a molten thermoplastic material through a plurality of fine, usually circular, die capillaries as molten threads or filaments into a high velocity, usually heated, gas (e.g., air) stream which attenuates the filaments of molten thermoplastic material to reduce their diameter. Thereafter, the meltblown fibers are carried by the high velocity gas stream and are deposited on a collecting surface to form a web of randomly disbursed meltblown fibers.

The terms "nonwoven" and "nonwoven web" refer to materials and webs of material having a structure of individual fibers or filaments which are interlaid, but not in an identifiable manner as in a knitted fabric. The terms "fiber" and "filament" are used herein interchangeably. Nonwoven fabrics or webs have been formed from many processes such as, for example, meltblowing processes, spunbonding processes, air laying processes, and bonded-carded-web processes. The basis weight of nonwoven fabrics is usually expressed in ounces of material per square yard (osy) or grams per square meter (gsm) and the fiber diameters are usually expressed in microns. (Note that to convert from osy to gsm, multiply osy by 33.91.)

The term "personal care articles" includes, but is not limited to, absorbent articles such as diapers, diaper pants, baby wipes, training pants, absorbent underpants, child care pants, swimwear, and other disposable garments; feminine care products including sanitary napkins, wipes, menstrual pads, menstrual pants, panty liners, panty shields, interlabials, tampons, and tampon applicators; adult-care products including wipes, pads such as breast pads, containers, incontinence products, and urinary shields; clothing components; bibs; athletic and recreation products; and the like.

The term "polymers" includes, but is not limited to, homopolymers, copolymers, such as for example, block, graft, random and alternating copolymers, terpolymers, etc. and blends and modifications thereof. Furthermore, unless otherwise specifically limited, the term "polymer" shall include all possible configurational isomers of the material. These configurations include, but are not limited to isotactic, syndiotactic and atactic symmetries.

The term "roll printing" or "roll coating" refers to a process in which the application of a deposited material, generally as a paste, onto a substrate is carried out by transferring the deposited material from a roll onto the substrate in a more or less uniform layer using one or more rolls, which may be engraved, and a pool cylinder. A doctor blade is used to scrape any excess deposited material from the rolls or substrate. The doctor blade may be flat or have a patterned edge such as slots or ridges.

The term "rotary screen printing" or "rotary screen coating" refers to a process that is a combination of roll printing or coating and screen printing or coating.

The term "screen printing" or "screen coating" refers to a method of applying a deposited material by forcing the material to be deposited through a screen that may have uniform openings or patterned openings.

The term "slot coating" refers to a process in which a slot die provides a thin, uniform coating on a substrate to be coated. In slot coating, the coating can be placed using an open gap in which the substrate to be coated is passed under the slot die, or a closed gap in which the slot die is aligned with a coating roll, such that there is a narrow gap or nip between the roller and slot die. The substrate to be coated is passed between the coating roll and the slot die.

The term "solution" when used in the phrase "flexible superabsorbent binder polymer solution," and derivatives thereof, refers to a polymer solution that has not yet been substantially crosslinked (i.e., a precursor), but will result in the flexible superabsorbent binder polymer composition once crosslinking occurs.

The term "spontaneous crosslinking" refers to crosslinking which occurs without radiation, catalysis, or any other inducement other than the specified temperature of not more than 150 °C, such as not more than 120 °C, or not more than 100 °C.

The terms "spunbond" and "spunbonded fiber" refer to fibers which are formed by extruding filaments of molten thermoplastic material from a plurality of fine, usually circular, capillaries of a spinneret, and then rapidly reducing the diameter of the extruded filaments.

The terms "superabsorbent" and "superabsorbent materials" refer to water-swellable, water-insoluble organic or inorganic materials capable, under the most favorable conditions, of absorbing at least 10 times their weight, or at least 15 times their weight, or at least 25 times their weight in an aqueous solution containing 0.9 weight percent sodium chloride. In contrast, "absorbent materials" are capable, under the most favorable conditions, of absorbing at least 5 times their weight of an aqueous solution containing 0.9 weight percent sodium chloride.

The term "unit" or "polymer unit" refers to a monomer or polymer portion of a copolymer molecule or blend component that includes a different molecular structure, compared to another portion of the copolymer or blend.

These terms may be defined with additional language in the remaining portions of the specification.

## DETAILED DESCRIPTION

The present invention concerns an absorbent article, such as a sanitary napkin or a wound-care article. More

particularly, the article comprises a backsheet and optionally a topsheet, and an absorbent layer that includes a flexible superabsorbent binder polymer composition and optionally a substrate. The absorbent layer can be in contact with a planar surface of the backsheet and/or the optional topsheet. Alternatively, the flexible superabsorbent binder polymer composition may be positioned between both the backsheet and a topsheet. In other aspects, the absorbent layer may function as the backsheet or as a topsheet.

The absorbent article of the present invention comprises a backsheet and an absorbent layer adjacent to and in facing relationship with the backsheet. The absorbent layer includes a flexible superabsorbent binder polymer composition formed by the reaction of a monomer solution including at least 15% by mass monoethylenically unsaturated monomer selected from carboxylic acid, carboxylic acid salts, sulphonic acid, sulphonic acid salts, phosphoric acid, or phosphoric acid salts; an acrylate or methacrylate ester that contains an alkoxysilane functionality; a plasticizer or a copolymerizable hydrophilic glycol containing an ester monomer; an initiator system; a chain transfer agent; a transition metal salt; and a neutralizing agent. The unsaturated monomer is neutralized to at least 25 mol%. The flexible superabsorbent binder polymer composition has a residual monoethylenically unsaturated monomer content of less than 1000 ppm. In some aspects of this embodiment, the absorbent layer can further comprise a substrate adjacent to and in facing relationship with the flexible superabsorbent binder polymer composition. In other aspects of this embodiment, the absorbent article can include a topsheet, where the topsheet is positioned such that the absorbent layer is positioned between the topsheet and the backsheet.

The flexible superabsorbent binder polymer composition has a weight average molecular weight of from 100,000 to 650,000 g/mole, and a viscosity after 16 hours of less than 10,000 cps. The flexible superabsorbent binder polymer composition has a residual monoethylenically unsaturated monomer content of less than 1000 ppm.

The result is an article which exhibits improved performance as well as greater comfort and confidence among the user. In some aspects, the flexible superabsorbent binder polymer composition can provide fluid adhesive properties in addition to retention properties. Thus, the flexible superabsorbent binder polymer composition is particularly suitable for use in forming absorbent products and/or laminated products. The term "flexible superabsorbent binder polymer composition" may also be referred herein as "composition," as "polymer composition," as "superabsorbent polymer composition," or as "absorbent material."

The articles of the present invention include, for example, personal care articles, health/medical articles, or household/industrial articles. Such articles are absorbent, and may further be disposable. Disposable absorbent articles include a backsheet, an optional topsheet which may be joined to the topsheet, and an absorbent layer positioned and held between the topsheet and the backsheet. The topsheet is operatively permeable to the fluids that are intended to be held or stored by the absorbent article, and the backsheet may or may not be substantially impervious or otherwise operatively impermeable to the intended fluids. The absorbent article may also include other components, such as fluid wicking layers, fluid intake layers, fluid distribution layers, transfer layers, barrier layers, wrapping layers and the like, as well as combinations thereof. Disposable absorbent articles and the components thereof can operate to provide a body-facing surface and an outward-facing surface. As used herein, a body-facing or bodyside surface means that the surface of the article or component which is intended to be disposed toward or placed adjacent to the body of the wearer during ordinary use, while the outward-facing surface is on the opposite side, and is intended to be disposed to face away from the wearer's body during ordinary use. Such outward surface may be arranged to face toward or placed adjacent to the wearer's undergarments when the absorbent article is worn.

The absorbent layer may comprise the flexible superabsorbent binder polymer composition alone, or may additionally include a substrate, as is shown in FIGS 1A and 1B, which are described in more detail below, or the absorbent material may be impregnated into the substrate, as is shown in FIG 1C. With reference to FIG 1A, which shows a cross-section of the absorbent layer of the present invention, the absorbent layer 10 has a substrate or support layer 11 and an absorbent layer 12. In one embodiment of the present invention, the absorbent layer may be formed on a surface of the substrate from an absorbent material 12'. As will be explained in more detail below, the absorbent material is prepared from a flexible superabsorbent binder polymer composition.

As is shown in FIG 1A, the absorbent layer 12 may be coextensive with the substrate 11. However, in the present invention, it is not necessary that the absorbent layer 12 is coextensive with the substrate layer 11. That is, the absorbent layer 12 does not completely cover the substrate 11 to the outer edges 99 of the substrate. In an alternative embodiment of the present invention shown in FIG 1B, the absorbent layer 12 containing the absorbent material 12' is not coextensive with the substrate 11, covering only a portion of the substrate 11 short of the outer edges 99 of the substrate 11. In another alternative embodiment of the present invention, the absorbent material may be placed within the substrate or impregnated into the substrate. This is shown in FIG 1C, where the absorbent material 12' is placed within the substrate 11. In order for the absorbent material to be impregnated or otherwise placed within the substrate 11, the substrate should be prepared from a material which contains interstitial spaces that allow the absorbent material 12' to penetrate the surface of the substrate 11 and allow the absorbent material 12' to be within interstitial spaces within the substrate 11. Whether the absorbent is a layer on the substrate or placed within the substrate, the substrate can act as a support layer, supporting the absorbent material, and the absorbent layer can be prepared on the substrate. It is

noted that the absorbent material 12' may appear to be shown in FIG 1C as a discrete phase or as discrete particles; however, the intent is to show that the flexible superabsorbent binder polymer composition 12' is impregnated into the substrate 11. Thus, the flexible superabsorbent binder polymer composition could be a continuous phase within the substrate.

The substrate of the absorbent layer may comprise a wide variety of materials. In addition, the absorbent layer can be fluid permeable or fluid impermeable. In some aspects, the substrate layer can be a film, a nonwoven web, a knitted fabric or a woven fabric, or a laminate of one or more of these materials. The only requirements for the substrate layer is that it has sufficient integrity so that the flexible superabsorbent binder polymer composition may be placed onto the substrate layer, or in the case of substrates with interstitial spaces, such as knitted fabrics, woven fabrics and nonwoven webs, or laminates containing these substrate materials, can be impregnated with the flexible superabsorbent binder polymer composition. In addition, the substrate should have sufficient flexibility so that the absorbent can be used in flexible absorbent articles. Particular examples of substrates include, but are not limited to, polyolefin films, spunbond nonwoven webs and laminates of polyolefin films and spunbond nonwoven webs, bonded-carded-webs, bonded-airlaid webs, coform, and woven fabrics such as cotton and wool cloth.

In general, as the thickness of the substrate increases, the integrity also increases. In some aspects, it may be desirable to have a very thin substrate to help increase flexibility and to help decrease the thickness of an absorbent article. However, if the thickness of the substrate is less that 0.01 mm, the substrate may be damaged during formation of the absorbent layer or during use in an absorbent article, unless the substrate is reinforced in some manner, for example, by laminating it to a nonwoven web. For example, in some aspects, when the substrate is a nonwoven web, the basis weight can be below about 100 gsm. However, in other aspects, the nonwoven substrate could have a basis weight in excess of 100 gsm. In some aspects, the basis weight of the nonwoven web should be between 7 gsm and 60 gsm, such as between 10 gsm and 40 gsm. In general, if the basis weight is below 7 gsm, the nonwoven web will tend to have insufficient strength to support the absorbent material.

In some aspects of the present invention, the flexible superabsorbent binder polymer composition may be placed directly on the substrate and can be directly joined or connected to the substrate, without the addition of adhesives, thereby forming a layer on the substrate. In other aspects, the flexible superabsorbent binder polymer composition can penetrate the substrate such that it will be impregnated into the support substrate. The flexible superabsorbent binder polymer composition may be applied to the substrate using any suitable application process, including slot coating, screen coating, knife over roll coating, or roll coating, either in a continuous coverage or a patterned coverage. Printing applications are other suitable application techniques, including gravure printing, screen, roll, rotary roll and jet printing. The flexible superabsorbent binder polymer composition may also be applied to the substrate using a spray application. The actual method of application of the flexible superabsorbent binder polymer composition to the optional substrate is not critical to the present invention. Once placed on the substrate, the flexible superabsorbent binder polymer composition is crosslinked, forming an absorbent coating on the substrate or forming a crosslinked absorbent material impregnated within the substrate.

To obtain a better understanding of the absorbent layer with additional substrate layers, attention is directed to FIGS 2A and 2B. FIG 2A shows an absorbent layer 10' having a substrate layer 11 impregnated with the flexible superabsorbent binder polymer composition 12' and an additional layer 13. The additional layer 13 is adjacent to the substrate 11 with the flexible superabsorbent binder polymer composition 12' impregnated therein. As is shown in FIG 2A, the substrate 11 with the flexible superabsorbent binder polymer composition 12' impregnated therein is coextensive with the side edges 99' of the additional layer 13. In an alternative embodiment, shown in FIG 2B, the substrate 11 having the flexible superabsorbent binder polymer composition 12' applied therein is positioned on the additional layer 13 such that the substrate and the flexible superabsorbent binder polymer composition therein is not coextensive with the edges 99 of the additional layer. As stated above in regard to FIG 1C, it is noted that the flexible superabsorbent binder polymer composition 12' may appear to be shown in FIGS 2A and 2B as a discrete phase or as discrete particles. However, the intent is to show that the flexible superabsorbent binder polymer composition 12' is impregnated within the substrate 11. That is, the flexible superabsorbent binder polymer composition 12' could be a continuous phase within the substrate 11.

In addition, the substrate 11 may have the flexible superabsorbent binder polymer composition 12' applied as an additional layer on the substrate 11, as is shown in FIG 2C. The additional layer 13 may be bonded to the substrate layer 11 using a known technique, such as adhesive bonding, pattern bonding using heat and pressure, ultrasonic bonding, stitching and other similar joining techniques. The layers of the absorbent layer may be held together using suitable bonding techniques, including those described above. In another aspect, the absorbent material 12 from the flexible superabsorbent binder polymer composition may adhesively hold the additional layer 13 to the substrate 11 as is shown in FIG 2D. When a three layer structure absorbent layer is desired or prepared, the flexible superabsorbent binder polymer composition may be applied to one of layers 11 or 13 or both layers 11 and 13. The layers are brought together so that the flexible superabsorbent binder polymer composition contacts each layer of the substrate 11 and additional layer 13 of the absorbent layer. As a result, the flexible superabsorbent binder polymer composition and the

resulting absorbent layer 12 are directly joined to the adjacent substrate 11 and additional layer 13, without an additional adhesive. This may be accomplished by applying the flexible superabsorbent binder polymer composition to facing surfaces of one or both layers 11 and 13, bringing the layers 11 and 13 together so that the flexible superabsorbent binder polymer composition contacts both layers, and crosslinking the flexible superabsorbent binder polymer composition to form the absorbent layer 12. In some aspects, crosslinking can be moisture-induced by hydrolysis and condensation of alkoxysilanes. For example, crosslinking of the flexible superabsorbent binder polymer composition can be induced by concentrating the composition through the removal of the water to promote condensation of silanols generated by hydrolysis of alkoxysilanes.

The flexible superabsorbent binder polymer composition layer may be formed on the substrate or support layer as a continuous layer having uniform thickness, or as a discontinuous or nonuniform layer which provides flow channels, fluid retention dams, or other desired attributes. However, because the absorbent layer 12 is intended as a sole or primary absorbent layer in the simplified absorbent article, the flexible superabsorbent binder polymer composition should be present in sufficient thickness and quantity, and over a sufficient area, to provide substantially all of the fluid absorption capacity that is required by the end use application. Alternatively, in some aspects, superabsorbent materials, such as superabsorbent particles, can be additionally incorporated into the absorbent binder to provide a portion of the liquid absorption capacity required by the end use application.

Because the flexible superabsorbent binder polymer composition is in contact with layers 11 and 13 as it is being formed, the resulting absorbent layer 12 adheres to the substrate layer and the additional layer 13 in addition to serving as an absorbent (fluid storage) layer. Thus, in some aspects of the present invention, the absorbent layer 10' can provide three layers bound together in sequence (i.e., a fluid receiving layer or backsheet, an absorbent layer, and a support layer) without intervening adhesive layers.

In other aspects, the flexible superabsorbent binder polymer composition may be prepared using a continuous process wherein the polymerization and/or neutralization reaction is carried out in a suitable reactor that conveys the resulting flexible superabsorbent binder polymer composition, upon completion of the polymerization reaction, directly to an apparatus for applying the composition onto the substrate layer 11 and/or the additional layer 13. Such a continuous process may be desirable where conditions, such as high heat, can cause premature crosslinking of the flexible superabsorbent binder polymer composition that would hinder application of the composition onto the substrate.

One advantage of the flexible superabsorbent binder polymer composition of the present invention is that it provides a water-soluble ionic polymer capable of sufficient spontaneous crosslinking within about 10 minutes, such as less than 5 minutes, or less than 1 minute, at a web temperature not more than 150°C, to provide the flexible absorbent binder layer with an absorbent capacity of at least one (1) gram of fluid per gram of flexible superabsorbent binder polymer composition, such as at least three (3) grams of fluid per gram of flexible superabsorbent binder polymer composition, using the Centrifuge Retention Capacity Test (described below).

The crosslinking at web temperatures not more than 150°C, such as not more than 120 °C, or not more than 100°C, permits the flexible superabsorbent binder polymer composition to be applied to one or more substrate layers, and then crosslinked, without degrading or damaging the substrate. Significant crosslinking occurs within about 10 minutes, such as within about 8 minutes, or within about 6 minutes to provide an efficient, commercially feasible, cost-effective crosslinking process. The crosslinking may then continue until a flexible superabsorbent binder polymer composition having the desired absorbent capacity is obtained. The ionic polymer may bear a positive charge, a negative charge, or a combination of both, and should have an ionic unit content of 15 mole % or greater. The ionic polymer may include a variety of monomer units described below.

In other aspects of the present invention, the absorbent layer 10" may have a second additional layer 14. In this regard, attention is directed to FIG 3A. The second additional layer 14 may be any of the same materials described above for the first additional layer 13. When the second additional layer is present, generally the substrate 11 and the flexible superabsorbent binder polymer composition 12' applied thereto are positioned between the first additional layer 13 and the second additional layer 14. Generally when two additional layers are present, one of the additional layers is a fluid impermeable material and the other additional layer is a fluid permeable material.

In other aspects of the present invention, the additional layer forms a backsheet for an absorbent article. The backsheet serves to prevent any fluids absorbed by the substrate 11 and the flexible superabsorbent binder polymer composition 12' applied thereon from passing through the absorbent article. Generally, the backsheet is fluid impermeable. In yet other aspects of the present invention, the second additional layer serves as a topsheet of for the absorbent article. The topsheet protects the substrate and the flexible superabsorbent binder polymer composition applied thereon during use of the absorbent article. In addition, the topsheet may serve to protect the user of the absorbent article from having direct contact with any superabsorbent material that may optionally be present in the flexible superabsorbent binder polymer composition 12'.

In still other aspects of the present invention, the absorbent layer 10' may have two distinct areas of the layer. To obtain a better understanding of this aspect of the present invention, attention is again directed to FIG 2B, which shows an absorbent layer 10' having a central region 97 and a perimeter region 98. The central region includes both

the additional layer 13, which is typically a backsheet, in which the substrate 11 and the flexible superabsorbent binder polymer composition 12' applied thereon is adjacent the additional layer 13 to form an absorbent article. The perimeter region 98 only includes the additional layer 13 or backsheet. In some aspects, this area is located in an insult target zone.

In yet another aspect of the present invention, as shown in FIG 3B, when the second additional layer is present, the second additional layer 14 (e.g., a topsheet) and the first additional layer 13 (e.g., a backsheet) are present in the perimeter region 98 and the substrate 11 with the flexible superabsorbent binder polymer composition 12' applied thereto along with the first and second additional layers 13, 14 are present in the central region. As stated above, in regard to FIG 1C, it is noted that the flexible superabsorbent binder polymer composition 12' may appear to be shown in FIGS 3A and 3B as a discrete phase or discrete particles. However the intent is to show that the flexible superabsorbent binder polymer composition 12' is impregnated within the substrate 11. That is, the absorbent material can be a continuous phase within the substrate 11.

In some aspects, the absorbent articles of the present invention can be relatively thin and can have a thickness in the range of 0.05 mm to 5 mm or more at a pressure of 1.35kPa. Generally, it is desirable that the absorbent articles be as thin as possible while providing sufficient absorbency. In some aspects, the absorbent articles of the present invention have a thickness in the range of 0.1 mm to 2.0 mm, such as 0.2 to 1.2 mm. In addition, the absorbent articles of the present invention can have an absorbency greater than 0.8 g/g, such as up to 10g/g of the absorbent layer, or 0.8 g/g to 5 g/g of the absorbent layer, as measured by the Centrifuge Retention Capacity Test.

The absorbent layer of the present invention also has the property of becoming soft and pliable under close-to-the-body conditions. The flexible superabsorbent binder polymer composition can be a very hydrophilic material with the ability to absorb water vapor. This property provides a benefit for thin absorbent articles because the relative stiffness of the article, when removed from the wrapper, allows the user to place the article in the undergarment with ease. However, when placed close to the body, the article becomes softer and more body conforming as a result of uptake of water vapor into the absorbent layer. This makes the absorbent layer of the present invention particularly suitable in absorbent articles, especially those absorbent articles utilized as sanitary napkins, pantiliners, diapers, bandages and the like.

The absorbent layer of the present invention can be used on its own or as an absorbent component or absorbent layer in a wide variety of absorbent articles including, but not limited to, personal care absorbent articles, household/ industrial absorbent articles and health/medical absorbent articles. In some aspects, the absorbent layer of the present invention may be particularly suited for use in sanitary napkins, pantiliners, bandages, bed liners, furniture liners as well as other absorbent articles, as described above. Typically, absorbent articles have an absorbent layer, and a backsheet, which helps retain any absorbed fluids in the absorbent article. Most absorbent articles have a backsheet which is a fluid impermeable layer. The backsheet generally faces away from the fluid source, meaning that the absorbent layer is positioned between the fluid source and the backsheet. In some applications, such as a bandage, the backsheet may be an apertured material, such as an apertured film, or material which is otherwise gas permeable, such as gas permeable films. In absorbent personal care articles such as pantiliners, the backsheet which is a fluid impermeable layer is often a garment facing layer. The backsheet is often referred to as a backing layer, baffle or outercover. Additional layers, such as a topsheet, also commonly referred to as a bodyside liner, may also be present in the absorbent article of the present invention.

The absorbent layer also includes a desired amount of the flexible superabsorbent binder polymer composition of the present invention. More specifically, the flexible superabsorbent binder polymer composition includes at least 15% by mass monoethylenically unsaturated carboxylic, sulphonic or phosphoric acid or salts thereof; an acrylate or methacrylate ester that contains an alkoxysilane functionality which, upon exposure to water, forms a silanol functional group which condenses to form a crosslinked polymer; a plasticizer or a copolymerizable hydrophilic glycol containing ester monomer; an initiator system; and a neutralizing agent. The polymer composition also includes a chain transfer agent, and a transition metal salt.

Suitable monomers that may be included to make a suitable superabsorbent polymer solution include carboxyl group-containing monomers, for example monoethylenically unsaturated mono or poly-carboxylic acids, such as (meth) acrylic acid (meaning acrylic acid or methacrylic acid; similar notations are used hereinafter), maleic acid, fumaric acid, crotonic acid, sorbic acid, itaconic acid, and cinnamic acid; carboxylic acid anhydride group-containing monomers, for example monoethylenically unsaturated polycarboxylic acid anhydrides (such as maleic anhydride); carboxylic acid salt-containing monomers, for example water-soluble salts (alkali metal salts, ammonium salts, amine salts, and the like) of monoethylenically unsaturated mono- or poly-carboxylic acids (such as sodium (meth)acrylate, trimethylamine (meth) acrylate, triethanolamine (meth)acrylate), sodium maleate, methylamine maleate; sulfonic acid group-containing monomers, for example aliphatic or aromatic vinyl sulfonic acids (such as vinylsulfonic acid, allyl sulfonic acid, vinyltoluenesulfonic acid, styrene sulfonic acid), (meth)acrylic sulfonic acids [such as sulfopropyl (meth)acrylate, 2 hydroxy-3-(meth)acryloxy propyl sulfonic acid]; sulfonic acid salt group-containing monomers, for example alkali metal salts, ammonium salts, amine salts of sulfonic acid group containing monomers as mentioned above; and/or amide group-containing monomers, for example vinylformamide, (meth)acrylamide, N-alkyl (meth)acrylamides (such as N-methylacrylamide, N-hexylacrylamide), N,N-dialkyl (meth)acryl amides (such as N,N-dimethylacrylamide, N,N-di-n-propylacrylamide), N hydroxyalkyl (meth)acrylamides [such as N-methylol (meth)acrylamide, N-hydroxyethyl (meth)acrylamide], N,N-dihydroxyalkyl (meth)acrylamides [such as N,N dihydroxyethyl (meth)acrylamide], vinyl lactams (such as N-vinylpyrrolidone).

Suitably, the amount of monoethylenically unsaturated carboxylic, sulphonic or phosphoric acid or salts thereof relative to the weight of the flexible superabsorbent binder polymer composition may range from 15% to 99.9% by weight. In some aspects, the levels of monoethylenically unsaturated carboxylic, sulphonic or phosphoric acid, or salts thereof, may be between 20% and 99.9% by weight of the flexible superabsorbent binder polymer composition, such as between 25% and 90% by weight of the flexible superabsorbent binder polymer composition, or between 30% and 80% by weight of the flexible superabsorbent binder polymer composition, or between 50% and 70% by weight of the flexible superabsorbent binder polymer composition for some intended uses.

The acid groups are neutralized to the extent of at least 25 mol%, that is, the acid groups are preferably present as sodium, potassium or ammonium salts. The degree of neutralization is preferably at least 50 mol%.

Organic monomers capable of co-polymerization with monoethylenically unsaturated carboxylic, sulphonic or phosphoric acid or salts thereof, which monomers contain a trialkoxysilane functional group or a moiety that reacts with water to form a silanol group, are useful in the practice of this invention. The trialkoxysilane functional group has the following structure:

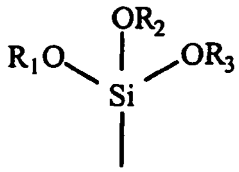

wherein R1, R2 and R3 are alkyl groups independently having from 1 to 6 carbon atoms.

The term "monomer(s)" as used herein includes monomers, oligomers, polymers, mixtures of monomers, and any other reactive chemical species which is capable of co-polymerization with monoethylenically unsaturated carboxylic, sulphonic or phosphoric acid or salts thereof. Ethylenically unsaturated monomers containing a trialkoxysilane functional group are appropriate for this invention and may be desired. Desired ethylenically unsaturated monomers include acrylates and methacrylates, such as acrylate or methacrylate esters that contain an alkoxysilane functionality. A particularly desirable ethylenically unsaturated monomer containing a trialkoxysilane functional group is methacryloxypropyl trimethoxy silane, commercially available from Dow Coming (having a place of business in Midland, Michigan, U.S.A.) under the trade designation Z-6030 SILANE and from Degussa (having a place of business in Parsippany, New Jersey, U.S.A) under the trade name DYNASYLAN MEMO. Other suitable ethylenically unsaturated monomers containing a trialkoxysilane functional group include, but are not limited to, methacryloxyethyl trimethoxy silane, methacryloxypropyl triethoxy silane, methacryloxypropyl tripropoxy silane, acryloxypropylmethyl dimethoxy silane, 3 acryloxypropyl trimethoxy silane, 3 methacryloxypropylmethyl diethoxy silane, 3 methacryloxypropylmethyl dimethoxy silane, and 3 methacryloxypropyl tris(methoxyethoxy)silane. However, it is contemplated that a wide range of vinyl and acrylic monomers having trialkoxysilane functional groups or a moiety that reacts easily with water to form a silanol group, such as a chlorosilane or an acetoxysilane, provide the desired effects and are effective monomers for copolymerization in accordance with the present invention.

Whereas most superabsorbent polymers require the addition of an internal crosslinker to reinforce the polymer, the flexible superabsorbent binder polymer composition of the present invention does not require the addition of a crosslinking agent because the organic monomers including the trialkoxysilane functional act as a latent internal crosslinker. The internal crosslinker allows the superabsorbent binder polymer composition to be formed by coating the water-soluble precursor polymer onto the substrate and then removing the water to activate the latent crosslinker.

In addition to monomers capable of co-polymerization that contain a trialkoxysilane functional group, a monomer capable of co-polymerization that can subsequently be reacted with a compound containing a trialkoxysilane functional group or a moiety that reacts with water to form a silanol group can also be used. Such a monomer may contain, but is not limited to, an amine or an alcohol. An amine group incorporated into the co-polymer may subsequently be reacted with, for example, but not limited to, (3-chloropropyl)trimethoxysilane. An alcohol group incorporated into the co-polymer may subsequently be reacted with, for example, but not limited to, tetramethoxysilane.

The amount of organic monomer having trialkoxysilane functional groups or silanol-forming functional groups relative to the weight of the polymeric binder composition may range from 0.1 % to 15% by weight. Suitably, the amount of monomer should exceed 0.1% by weight in order to provide sufficient crosslinking upon exposure to moisture. In some aspects, the monomer addition levels are between 0.1% and 20% by weight of the flexible superabsorbent binder polymer composition, such as between 0.5% and 10% by weight of the flexible superabsorbent binder polymer composition, or between 0.5% and 5% by weight of the flexible superabsorbent binder polymer composition for some intended uses.

The flexible superabsorbent binder polymer composition can include a copolymerizable hydrophilic glycol containing an ester monomer, for example long chain, hydrophilic monoethylenically unsaturated esters, such as poly (ethylene glycol) methacrylate having from 1 to 13 ethylene glycol units. The hydrophilic monoethylenically unsaturated esters have the following structure:

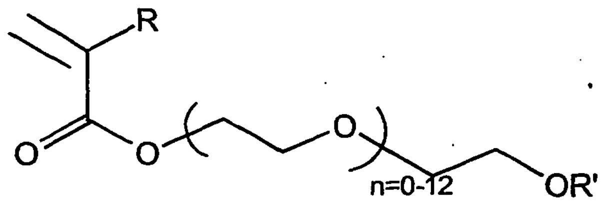

R'= H, alkyl, phenyl

R= H or $CH_3$

The amount of monoethylenically unsaturated hydrophilic esters relative to the weight of the polymeric binder composition thereof may range from 0 to 75% by weight of monomer to the weight of the flexible superabsorbent binder polymer composition. In some aspects, the monomer addition levels are between 10% and 60% by weight of the flexible superabsorbent binder polymer composition; such as between 20% and 50% by weight of the flexible superabsorbent binder polymer composition, or between 30% and 40% by weight of the flexible superabsorbent binder polymer composition, for some intended uses.

In some aspects, the flexible superabsorbent binder polymer composition may also include a plasticizer, such as a hydrophilic plasticizer. Suitable hydrophilic plasticizers include, but are not limited to, polyhydroxy organic compounds such as glycerin and low molecular weight polyolefinic glycols such as polyethylene glycol (PEG) of molecular weight ranges from 200 to 10,000.

The amount of plasticizer relative to the weight of the flexible superabsorbent binder polymer composition thereof may range from 0 to 75% by weight of the plasticizer to the weight of the flexible superabsorbent binder polymer composition. In some aspects, the plasticizer addition levels are from 10% to 60% by weight of the flexible superabsorbent binder polymer composition, such as from about 10% to 40% by weight of the flexible superabsorbent binder polymer composition, for some intended uses.

In some aspects, the flexible superabsorbent binder polymer composition of the present invention may be made from monomers that include at least 15% by weight monoethylenically unsaturated monomer selected from carboxylic acid, carboxylic acid salts, sulphonic acid, sulphonic acid salts, phosphoric acid, or phosphoric acid salts; an initiator system; and an acrylate or methacrylate ester that contains a group readily transformed into a silanol functionality by subsequent reaction with water, wherein the resulting flexible superabsorbent binder polymer composition has an average molecular weight of from 100,000 to 650,000 g/mole, such as 100,000 to 300,000 g/mole, nd the superabsorbent polymer composition has a viscosity of less than 10,000 cps and a residual monoethylenically unsaturated monomer content of less than 1000 ppm.

The superabsorbent polymer composition may be prepared by adding a solution of the above monomers to an initiator system, at a suitable temperature, to generate free radicals, for example between 50 °C and 90 °C. An initiator system may be prepared by dissolving an initiator in a solvent. Initiators are used to start the polymerization of a monomer. The action of an initiator is similar to that of a catalyst, except that the initiator is generally consumed in the reaction. Possible solvents include, but are not limited to water, and alcohols such as ethanol. A variety of initiators may be useful in the practice of this invention. The polymerization initiator system may be activated using a variety of methods including, but not limited to, thermal energy, radiation, redox chemical reactions, thermal initiators and other methods known in the art. One suitable class of initiators is organic peroxides and azo compounds, with benzoyl peroxide and azobisisobutyronitrile (AIBN), as examples. Examples of suitable initiators include t-amylperoxypivalate, 2,2'-Azobis(2,4'-dimethylvaleronitrile) (V65B), sodium persulfate (NAPS); and 2,2'-azobis-2-amidinopropanedihydrchloride (ABAH). Suitable amounts of initiators depend upon the particular initiator. Examples include, but are not limited to, at least about 0.003 mol/mol of t-amylperoxypivalate; at least 0.01 mol/mol of 2,2'-Azobis(2,4'-dimethylvaleronitrile); at least 200 ppm of sodium persulfate; and at least 200ppm of 2,2'-azobis-2-amidinopropanedihydrchloride.

Compounds containing an O-O, S--S, or N = N bond may be used as thermal initiators. Compounds containing O-O bonds, such as peroxides, are commonly used as initiators for polymerization. Examples of peroxide initiators include alkyl, dialkyl, diaryl and arylalkyl peroxides such as cumyl peroxide, t-butyl peroxide, di-t-butyl peroxide, dicumyl

peroxide, cumyl butyl peroxide, 1,1-dit-butyl peroxy-3,5,5 trimethylcyclohexane, 2,5-dimethyl-2,5-di(t-butylperoxy)hexane, 2,5-dimethyl-2,5 bis(t-butylperoxy)hexyne-3 and bis(a-t-butyl peroxyisopropylbenzene); acyl peroxides such as acetyl peroxides and benzoyl peroxides; hydroperoxides such as cumyl hydroperoxide, t-butyl hydroperoxide, p-methane hydroperoxide, pinane hydroperoxide and cumene hydroperoxide; peresters or peroxyesters such as t-butyl peroxypivalate, t amylperoxypivalate, t-butyl peroctoate, t-butyl perbenzoate, 2,5-dimethylhexyl-2,5 di(perbenzoate) and t-butyl di(perphthalate); alkylsulfonyl peroxides; dialkyl peroxymonocarbonates; dialkyl peroxydicarbonates; sodium persulfate, 2,2'-Azobis(2,4'-dimethylvaleronitrile), 2,2'-azobis-2-amidinopropanedihydrchloride.diperoxyketals; and ketone peroxides such as cyclohexanone peroxide and methyl ethyl ketone peroxide. In one particular aspect of the present invention, an organic initiator, t-amylperoxypivalate (TAPP) that decomposes very fast to form a stable ethyl (CH3CH2•) free radical was utilized to reduce the residual monoethylenically unsaturated monomer significantly.

A redox initiator system where free radicals are generated by oxidation-reduction reactions without the application of heat can be used for the polymerization of the monomer solution to make the flexible superabsorbent binder polymer composition. In this method, polymerization is started by adding either one of oxidizing or reducing components of the initiator system to the rest of the solution mixture of monomers and other components of the redox initiator system. Suitable oxidizing components of the redox initiator system include, but are not limited to, hydrogen peroxide, alkali metal persulfates, ammonium persulfate, alkalihydroperoxides, peresters, diacryl peroxides, silver salts and combinations thereof. Suitable reducing components of the initiator system include, but are not limited to, ascorbic acid, alkali metal sulfites, alkali metal bisulfites, ammonium sulfite, ammonium bisufite, alkali metal hydrogen sulfites, ferrous metal salts such as ferrous sulfates, sugars, aldehydes, primary and secondary alcohols, and combinations thereof. A combination of redox and thermal initiators can also be used. A redox initiator system that comprises hydrogen peroxide, ferrous sulfate and ascorbic acid coupled with thermal initiator sodium persulfate (NAPS) was found to reduce the residual monoethylenically unsaturated monomer significantly in aqueous polymerization of the present invention while yielding a weight average molecular weight of superabsorbent polymer in the target range of 100,000 to 650,000 g/mole, such as 1000,000 to 300,000 g/mole.

A chain transfer agent that can limit the polymer chain growth during the polymerization and thereby can control the molecular weight and viscosity of flexible superabsorbent binder polymer solution is used in the polymerization solution. Suitable chain transfer agents include, but are not limited to, alcohols such as isopropyl alcohol, organic acids such as formic acid, inorganic acids such as hypophosphorus acid, organic amines such as triethylamine and combinations thereof. In one aspect, hypophosphorus acid was found to be an effective chain transfer agent for the flexible superabsorbent binder polymer composition.

The amount of chain transfer agent relative to the weight of monoethylenically unsaturated carboxylic, sulphonic or phosphoric acid or salts monomers may range from 0.1 to 20% by weight of the chain transfer agent to the weight of the monomers. In some aspects, the chain transfer agent addition levels can be between 5% and 15% by weight of the monomer, such as between 2% and 10% by weight of the monomer, or between 0.5% and 1% by weight of the monomer to obtain desired molecular weight and viscosity levels of the flexible superabsorbent binder polymer composition, for some intended uses.

The method to make the flexible superabsorbent polymer composition of the present invention further includes a transition metal salt. Examples of some suitable transition metals for the transition metal salt include, but are not limited to, scandium, titanium, vanadium, chromium, manganese, iron, cobalt, nickel, copper, zinc, silver, and the like. For instance, a transition metal salt may be combined with the flexible superabsorbent binder polymer compositions before, during, and/or after its formation. For instance, some transition metal salts that may be used in the present invention include, but are not limited to, halides, such as iron chloride, copper chloride, sulfates, nitrates, acetates, oxalates, carbonates, and so forth. Iron sulfate may be used in the present invention.

In some aspects of the present invention, a new source of crosslinking having silanol functionality may be added to the superabsorbent binder polymer composition just before the concentrated solution is applied to a substrate. The added source of silanol crosslinking functionality may, in effect, activate the solution for gellation as it is being applied to the substrate. Potential sources of silanol (Si-OH) functionality include, but are not limited to silica nano particles, such as SNOWTEX ST-40 (available from Nissan Chemical - America Corporation, having a place of business located in Houston, Texas, U.S.A.); silica aerogel particles, such as SYLOID silica (available from Grace Davison, a division of W.R. Grace & Co., having a place of business in Columbia. Maryland, U.S.A.); clays with Si-OH surface, such as Kaolin, bentonite, or attapolgite; and zeolites. In addition, soluble sources of silanol can be added, such as silicates, or in the form of monomeric silanes that are readily hydrolyzed to silanols, such as, but not limited to alkoxysilanes, for example, tetraethoxy silane (TEOS).

The source of silanol can be added in any suitable manner to provide sufficient mixing with the flexible superabsorbent binder polymer composition solution prior to coating onto the substrate. For example, two separate, metered, feed streams of the flexible superabsorbent binder polymer solution and silanol source may be combined at a Y-juncture with a down-stream static mixer in the flow line to provide mixing.

Suitable ranges may be any that provide for a stable solution at a polymer concentration greater than 25%.

Alkoxysilane functionality is incorporated into a base flexible superabsorbent binder polymer solution at an acrylate to silane mole ratio of 170:1. Flexible superabsorbent binder polymer composition with 75%, 50% and 25% of a base flexible superabsorbent binder polymer solution incorporation have been prepared. (Mole ratios of acrylate to silane in these polymers are 227:1, 340:1, and 680:1). Table 1 below shows the absorbent capacity data, based on the Centrifuge Retention Capacity Test (described below):

Table 1

| **Polymer composition** | CRC g/g |
|---|---|
| Standard composition: acrylate : Si-OH ratio 56:1 | 14.2 |
| 50% reduced alkoxysilane: acrylate to Si-OH ratio 112:1 | 21.4 |
| 50% reduced alkoxysilane: with kaolin added to reduce acrylate to Si-OH ratio to 20:1 | 15.1 |
| 50% reduced alkoxysilane: with syloid silica added to reduce acrylate to Si-OH ratio to 20:1 | 14.6 |
| 50% reduced alkoxysilane: with tetraethoxy silane added to reduce acrylate to Si-OH ratio to 20:1 | 17.1 |

As demonstrated in Table 1, reducing the alkoxysilane incorporation increased the Centrifuge Retention Capacity (CRC) due to lower crosslink density, compared to a base flexible superabsorbent binder polymer composition. Addition of sources of silanol, even to levels greater than a base flexible superabsorbent binder polymer composition, provides a higher CRC than a base flexible superabsorbent binder polymer composition, even with higher crosslinking potential. Once the flexible superabsorbent binder polymer composition is applied to the substrate, crosslinking can be moisture-induced by hydrolysis and condensation of alkoxysilanes. Activation by this method can take place during solvent removal or after solvent removal by exposure to air at ambient humidity. Solvent may be removed from the substrate either by evaporating the solvent or by any other suitable technique. Heat or radiation may be applied to increase the rate of the process. Recovery of the solvent is a part of the process and methods for this are widely known to those skilled in the art.

In addition, modifying agents such as compatible polymers, plasticizers, colorants, and preservatives may be incorporated in the flexible superabsorbent binder polymer composition of the present invention.

In some aspects of the present invention, the flexible superabsorbent binder polymer composition of the present invention may be prepared in an aqueous solution by the process including the steps of: a) preparing an initiator system solution; b) preparing a monomer solution including monoethylenically unsaturated monomers, one of which includes an alkyloxysilane functionality; c) mixing the initiator system and the monomer solution to form a polymerization solution; d) heating the polymerization solution to promote a reaction of the polymerization solution; e) cooling the polymerization solution; and f) neutralizing the polymer of step e) to at least 25 mole% to form a flexible superabsorbent binder polymer composition, the dry polymer of which has a residual monoethylenically unsaturated monomer content less than 1000 ppm. In addition, the polymer composition has a weight average molecular weight of from 100,000 to 650,000 g/mole, such as from 100,000 to 300,000 g/mole, and a viscosity after 16 hours of less than 10,000 cps. Furthermore, the flexible superabsorbent binder polymer composition may have a solids content of at least 24% by weight.

In other aspects of the present invention, the flexible superabsorbent binder polymer composition of the present invention may be prepared in an aqueous solution by the process including the steps of: a) preparing a monomer solution including an initiator system that includes one component of a redox initiator; a chain transfer agent; a plasticizer; a cross-linker monomer that contains an alkoxysilane functionality; and monoethylenically unsaturated monomers, one of which includes a functionality wherein the acid groups are neutralized to at least 25 mole%; b) adding another component of the redox initiator to the monomer solution mixture of step a) to polymerize the monomer solution mixture of a); c) cooling the polymerization solution to a temperature less than 30°C; d) adding a similar solution mixture of step a) to the polymerization solution of step c); e) adding a transition metal salt to the solution of step d); f) polymerizing the solution of step e); and g) optionally neutralizing the polymer of step f) to form a flexible superabsorbent binder polymer composition having a average molecular weight of from 100,000 to 650,000 g/mole, such as from 100,000 to 300,000 g/mole, a viscosity after 16 hours of less than 10,000 cps and a residual monoethylenically unsaturated monomer content of less than about 1000 ppm. Furthermore, the flexible superabsorbent binder polymer composition may have a solids content of at least 24% by weight.

In still other aspects of the present invention, the flexible superabsorbent binder polymer composition of the present invention may be prepared in an aqueous solution by the process including the steps of: a) preparing a monomer solution including an initiator system, a chain transfer agent, an initiating system, a plasticizer, a cross-linker monomer that contains an alkoxysilane functionality, monoethylenically unsaturated monomers, one of which includes a functionality wherein the acid groups are neutralized to at least 25 mole%; b) polymerizing the monomer solution mixture of step

a) to promote a polymerization reaction; c) cooling the polymerization solution to a temperature of less than 30°C; d) adding a second monomer solution, neutralizing agent and plasticizer to the polymerization solution of step c); e) adding a transition metal salt to the solution of step d); f) further reacting the solution of step e); and g) optionally, further neutralizing the polymer of step f) to form a flexible superabsorbent binder polymer composition having a weight average molecular weight of from 100,000 to 650,000 g/mole, such as 100,000 to 300,000 g/mole, a viscosity after 16 hours of less than 10,000 cps and a residual monoethylenically unsaturated monomer content of less than 1000 ppm. Furthermore, the flexible superabsorbent binder polymer composition may have a solids content of at least 24% by weight.

In the present invention, the absorbent layer of the absorbent article can be prepared as described above. In particular the absorbent layer may include only the flexible superabsorbent blinder polymer compositions of the present invention, or it may be a substrate having the flexible superabsorbent binder polymer composition, described above, applied to the substrate. Also as described above, additional layers of the absorbent layer of the present invention may also function as a layer of the absorbent article. For example, if the substrate layer 11 is a fluid impermeable material, the substrate layer of the absorbent layer could also function as the fluid impermeable layer of the absorbent article, for example, the backsheet of the absorbent article. To obtain a better understanding of an absorbent article of the present invention, attention is directed to FIG 4.

FIG 4 illustrates an example of a suitable article, such as the representatively shown feminine care article 20, which is configured to incorporate the present invention. It is understood that the present invention is suitable for use with various other articles, including but not limited to other personal care articles, health/medical articles, household/ industrial articles, and the like, without departing from the scope of the present invention.

The article can comprise an absorbent body structure, and the absorbent body can include an absorbent layer 30 which may include the flexible superabsorbent binder polymer composition of the present invention only. In other aspects, the absorbent layer 30 may also include a substrate. In yet other aspects, the absorbent layer 30 may also include additional components, such as wood fibers or other superabsorbent materials. For example, the absorbent layer 30 may comprise the flexible superabsorbent binder polymer composition of the present invention operatively contained within or in contact with a matrix of fibers.

Desirably, the substrate can include an operative amount of fluff and/or polymer fibers. In some aspects, the layer 30 can include at least 60% by weight of the flexible superabsorbent binder polymer composition, and not more than 40% by weight of the fibers, based on a total weight of the composite.

The article 20 can have a lengthwise longitudinal direction 22, a transverse, laterally extending, cross-direction 24, first and second longitudinally opposed end portions 72 and 72a, and an intermediate portion 76 located between the end portions. As representatively shown, the longitudinal dimension of the article is relatively larger than the lateral dimension of the article. The article 20 can include a topsheet 26, a backsheet 28, and the absorbent layer 30 positioned between the topsheet and backsheet. The absorbent layer 30 can have configurations which are selectively constructed and arranged to provide desired performance and aesthetics.

By incorporating its various features, aspects and configurations, alone or in desired combinations, the article can provide an improved absorbent system that can take better advantage of the functional properties of the flexible superabsorbent binder polymer composition of the present invention. For example, the article can provide comparable or improved absorbent properties when compared to an article comprising a conventional absorbent layer. Similarly, the article can provide comparable or improved absorbent properties while reducing the thickness and/or improving the flexibility of the article, as well as improved production costs, when compared to an article comprising a conventional absorbent layer.

In some aspects, the absorbent layer 30 can also distribute viscous fluids more efficiently to desired locations in an absorbent article, as well as provide a drier body-facing surface, and in particular configurations, can provide visual cues of absorbency. Other examples can provide improved appearance and aesthetics. As a result, an article incorporating the invention can provide greater comfort and fit, and can improve protection and increase confidence.

The optional topsheet 26 of the article 20 may include a layer constructed of any operative material, and may be a composite material. For example, the topsheet layer can include a woven fabric, a nonwoven fabric, a polymer film, a film-fabric laminate or the like, as well as combinations thereof. Examples of a nonwoven fabric include spunbond fabric, meltblown fabric, coform fabric, carded-web, bonded-carded-web, bicomponent spunbond fabric or the like, as well as combinations thereof. Other examples of suitable materials for constructing the topsheet layer can include rayon, bonded-carded-webs of polyester, polypropylene, polyethylene, nylon, or other heat-bondable fibers, polyolefins, such as copolymers of polypropylene and polyethylene, linear low-density polyethylene, biodegradable aliphatic polyesters such as poly(hydroxyl alkanoates) and polyactic acid, finely perforated film webs, net materials, and the like, as well as combinations thereof.

A more particular example of a suitable topsheet layer material can include a bonded-carded-web composed of polypropylene and polyethylene, such as has been used as a topsheet stock for KOTEX brand pantiliners, and has been obtainable from Vliesstoffwerk Christian Heinrich Sandler GmbH & Co. KG, a business having an address at Postfach 1144, D95120 Schwarzenbach/Saale, Germany. Other examples of suitable materials are composite materials

of a polymer and a nonwoven fabric material. The composite materials are typically in the form of integral sheets generally formed by the extrusion of a polymer onto a web of spunbond material. In a desired arrangement, the topsheet layer 26 can be configured to be operatively fluid-permeable with regard to the fluids that the article is intended to absorb or otherwise handle. The operative fluid-permeability may, for example, be provided by a plurality of pores, apertures or other openings, as well as combinations thereof, that are present or formed in the topsheet layer. The apertures or other openings can help increase the rate at which bodily fluids can move through the thickness of the topsheet layer and penetrate into the other components of the article (e.g., the absorbent layer 30). The selected arrangement of fluid-permeability is suitably present at least on an operative portion of the topsheet layer that is appointed for placement on the body-side of the article. The topsheet 26 can provide comfort and conformability, and can function to direct complex fluids, such as menses, away from the body and toward the absorbent layer 30. In one example, the topsheet layer 26 can be configured to retain little or no liquid in its structure, and can be configured to provide a relatively comfortable and non-irritating surface next to the body-tissues of the wearer. The topsheet layer 26 can be constructed of any material which is also easily penetrated by viscous or complex fluids that contact the surface of the topsheet layer.

The optional topsheet 26 can have at least a portion of its bodyside surface treated with a surfactant to render the topsheet more hydrophilic. The surfactant can permit arriving viscous liquids to more readily penetrate the topsheet layer. The surfactant may also diminish the likelihood that the arriving fluids will flow off the topsheet layer rather than penetrate through the topsheet layer into other components of the article. In one example, the surfactant can be substantially evenly distributed across at least a portion of the upper, bodyside surface of the topsheet 26 that overlays the body-facing surface of the absorbent layer 30.

The optional topsheet 26 may be maintained in secured relation with the absorbent layer 30 of the present invention by bonding all or a portion of the adjacent surfaces to a surface of the absorbent layer 30, the backsheet 28, or both. A variety of bonding techniques known to one of skill in the art may be utilized to achieve any such secured relation. Examples of such techniques include, but are not limited to, the application of adhesives in a variety of patterns between the two adjoining surfaces, entangling at least portions of the adjacent surface of the absorbent layer 30 and/or backsheet 28 with portions of the adjacent surface of the topsheet 26, co-aperturing or fusing at least portions of the adjacent surface of the topsheet 26 to portions of the adjacent surface of the absorbent layer 30 and/or backsheet 28. The topsheet 26 may also be held in secured relation to the absorbent layer 30 through the adhesive properties exhibited by the flexible superabsorbent binder polymer composition of the present invention.

The optional topsheet 26 typically extends-over the body-facing surface of the absorbent layer 30, but can alternatively extend around the article to partially, or entirely, surround or enclose the composite. Alternatively, the topsheet 26 and the backsheet 28 can have peripheral margins which extend outwardly beyond the terminal, peripheral edges of the absorbent layer 30, and the extending margins can be joined together to partially, or entirely, surround or enclose the composite. In some aspects, the flexible superabsorbent binder polymer composition of the present invention can function as the topsheet, in addition to functioning as or in an absorbent layer. Thus the flexible superabsorbent binder polymer composition can also provide an innermost bodyside surface.

The backsheet 28 may include a layer constructed of any operative material, and may or may not have a selected level of fluid-permeability or fluid-impermeability, as desired. In one example, the backsheet 28 may be configured to provide an operatively fluid-impermeable structure. The backsheet 28 may, for example, include a polymeric film, a woven fabric, a nonwoven fabric or the like, as well as combinations or composites thereof. For example, the backsheet 28 may include a polymer film laminated to a woven or nonwoven fabric. In some aspects, the polymer film can be composed of polyethylene, polypropylene, polyester or the like, as well as combinations thereof. Additionally, the polymer film may be micro-embossed, have a printed design, have a printed message to the consumer, and/or may be at least partially colored. Suitably, the backsheet 28 can operatively permit a sufficient passage of air and moisture vapor out of the article, particularly out of the absorbent layer 30, while blocking the passage of bodily fluids.

An example of a suitable backsheet material can include a breathable, microporous film, such as a HANJIN Breathable Backsheet available from Hanjin Printing, Hanjin P&C Company Limited, a business having offices located in Sahvon-li.Jungan-mvu.Kongiu-City, Chung cheong nam-do, Republic of South Korea. This backsheet material is a breathable film, which is white in color, dimple embossed, and contains: 47.78% calcium carbonate, 2.22% $TiO_2$, and 30% polyethylene.

In one example, the polymer film can have a minimum thickness of no less than 0.025 mm, and in another feature, the polymer film can have a maximum thickness of no greater than 0.13 mm. Bicomponent films or other multicomponent films can also be used, as well as woven and/or nonwoven fabrics which have been treated to render them operatively fluid-impermeable. Another suitable backsheet material can include a closed-cell polyolefin foam. For example, a closed-cell polyethylene foam may be employed. Still another example of a backsheet material would be a material that is similar to a polyethylene film which is used on currently commercially sold KOTEX brand pantiliners, and is obtainable from Pliant Corporation, having a place of business in Schaumburg, Illinois, U.S.A. In some aspects, the flexible superabsorbent binder polymer composition of the present invention can function as the backsheet, in addition to functioning as or in an absorbent layer. Thus, the flexible superabsorbent binder polymer composition can provide

an outermost garment-facing surface.

The absorbent layer 30 comprising the flexible superabsorbent binder polymer composition of the present invention can be sized and placed to more effectively operate in an insult target zone of the absorbent article 20 where fluids are more likely to be introduced into the article. The structure of the absorbent layer 30 can be operatively configured to provide a desired level of fluid acquisition, distribution and retention. The absorbent layer 30 may include one or more components that can modify the composition or rheological properties of such viscous fluids. In some aspects, the absorbent layer 30 may additionally or alternatively include materials such as surfactants, ion exchange resin particles, moisturizers, emollients, perfumes, natural fibers, synthetic fibers, fluid modifiers, odor control additives, and combinations thereof. Alternatively, the absorbent layer 30 can include a foam.

In order to function well, the absorbent layer 30 can have certain desired properties to provide improved performance as well as greater comfort and confidence among the user. For instance, the absorbent layer 30 can have corresponding configurations of absorbent capacities, densities, basis weights and/or sizes which are selectively constructed and arranged to provide desired combinations of absorbency properties such as fluid intake rate, absorbent capacity, fluid distribution or fit properties such as shape maintenance and aesthetics. Likewise, the components can have desired wet to dry strength ratios, mean flow pore sizes, and permeabilities.

The optional substrate of the absorbent layer 30 can include an amount of a surfactant. The surfactant can be combined with the substrate of the absorbent layer in any operative manner. Various techniques for combining the surfactant are conventional and well known to persons skilled in the art. For example, the surfactant may be compounded with polymer employed to form a meltblown fiber structure. In a particular feature, the surfactant may be configured to operatively migrate or segregate to the outer surface of the fibers upon the cooling of the fibers. Alternatively, the surfactant may be applied to or otherwise combined with spunbond fibers after the fibers have been formed.

The substrate can include an operative amount of surfactant, based on the total weight of the substrate and surfactant. In some aspects, the substrate can include at least a minimum of 0.1 % by weight surfactant, as determined by water extraction. The amount of surfactant can alternatively be at least 0.15% by weight, and can optionally be at least 0.2% by weight to provide desired benefits. In other aspects, the amount of surfactant can be generally not more than a maximum of 2% by weight, such as not more than 1 % by weight, or not more than 0.5% by weight to provide improved performance.

In some configurations, the surfactant can include at least one material selected from the group that includes polyethylene glycol ester condensates and alkyl glycoside surfactants. For example, the surfactant can be a GLUCOPON surfactant, available from Cognis Corporation (having a place of business in Cincinnati, Ohio, U.S.A.) which can be composed of 40% by weight water, and 60% by weight d-glucose, decyl, octyl ethers and oligomerics.

The absorbent article may also include other components, such as fluid wicking layers, intake layers, surge layers, distribution layers, transfer layers, barrier layers, wrapping layers and the like, as well as combinations thereof. For example, with reference to FIG 4, a surge layer 32 in one aspect may be positioned between the topsheet 26 and the absorbent layer 30.

In other aspects of the invention, the surfactant can be in the form of a sprayed-on surfactant comprising a water/surfactant solution which includes 16 liters of hot water (45 °C to 50 °C) mixed with 0.20 kg of GLUCOPON 220 UP surfactant available from Cognis Corporation and 0.36 kg of AHCHOVEL Base N-62 surfactant available from Uniqema (having a place of business in New Castle, Delaware, U.S.A.).

In addition to the absorbent articles described above, the absorbent articles of the present invention may be used as an absorbent bandage. Attention is directed to FIGS 5A and 5B, which show a possible configuration for a bandage of the present invention. FIG 5A shows a cross-section view of the absorbent bandage with optional layers described below. FIG 5B shows a perspective view of the bandage of the present invention with some of the optional or removable layers not being shown. The absorbent bandage 70 has a strip 71 of material having a body-facing side 79 and a second side 78 which is opposite the body-facing side. The strip is essentially a backsheet and is desirably prepared from the same materials described above for the backsheet. In addition, the strip may be apertured material, such as an apertured film, or material which is otherwise gas permeable, such as a gas permeable film. The strip 71 supports an absorbent layer 72 which is attached to the body facing side 79 of the strip. In addition, an optional absorbent protective layer 73 may be applied to the absorbent layer 72 and can be coextensive with the strip 71. The absorbent layer 72 contains the flexible superabsorbent binder polymer composition of the present invention.

The absorbent bandage 70 of the present invention may also have a pressure sensitive adhesive 74 applied to the body-facing side 79 of the strip 71. Any pressure sensitive adhesive may be used, provided that the pressure sensitive adhesive does not irritate the skin of the user. Suitably, the pressure sensitive adhesive is a conventional pressure sensitive adhesive which is currently used on similar conventional bandages. This pressure sensitive adhesive is preferably not placed on the absorbent layer 72 or on the absorbent protective layer 73 in the area of the absorbent layer 72. If the absorbent protective layer is coextensive with the strip 71, then the adhesive may be applied to areas of the absorbent protective layer 73 where the absorbent layer 72 is not located. By having the pressure sensitive adhesive on the strip 71, the bandage is allowed to be secured to the skin of a user in need of the bandage. To protect the pressure

sensitive adhesive and the absorbent, a release strip 75 can be placed on the body facing side 79 of the bandage. The release liner may be removably secured to the article attachment adhesive and serves to prevent premature contamination of the adhesive before the absorbent article is secured to, for example, the skin. The release liner may be placed on the body facing side of the bandage in a single piece (not shown) or in multiple pieces, as is shown in FIG 5A.

In another aspect of the present invention, the absorbent layer of the bandage may be placed between a folded strip. If this method is used to form the bandage, the strip is suitably fluid permeable.

Absorbent furniture and/or bed pads or liners are also included within the present invention. As is shown in FIG 6, a furniture or bed pad or liner 80 (hereinafter referred to as a "pad") is shown in perspective. The pad 80 has a fluid impermeable backsheet 81 having a furniture-facing side or surface 88 and an upward facing side or surface 89 which is opposite the furniture-facing side or surface 88. The fluid impermeable backsheet 81 supports an absorbent layer 82 which is attached to the upward facing side 89 of the fluid impermeable backsheet. In addition, an optional absorbent protective layer 83 may be applied to the absorbent layer. The absorbent layer contains the flexible superabsorbent binder polymer composition of the present invention. The optional substrate layer of the absorbent layer can be the fluid impermeable layer 81 or the absorbent protective layer 83 of the pad. In the alternative, in aspects where the absorbent layer has three layers, the three layers of the absorbent layer can include the fluid impermeable layer 81, the flexible superabsorbent binder polymer composition layer 82 and the absorbent protective layer 83.

To hold the pad in place, the furniture-facing side 88 of the pad may contain a pressure sensitive adhesive, a high friction coating or other suitable material which will aid in keeping the pad in place during use. The pad of the present invention can be used in a wide variety of applications including placement on chairs, sofas, beds, car seats and the like to absorb any fluid which may come into contact with the pad.

The present invention may be better understood with reference to the following examples.

## TEST PROCEDURES

### <u>Residual Monoethylenically Unsaturated Monomer Test</u>

The residual monoethylenically unsaturated monomer analysis is carried out using a solid film obtained from the superabsorbent polymer solution. By way of example for this test description, the monoethylenically unsaturated monomer is acrylic acid. High performance liquid chromatography (HPLC) with a SPD-10Avp SHIMADZU UV detector (Shimadzu Scientific Instruments, 7102 Riverwood Drive, Columbia, Maryland, U.S.A) is used to determine the residual acrylic acid monomer content. To determine the residual acrylic acid monomer, approximately 0.5 grams of cured film is stirred in 100 ml of a 0.9% NaCl-solution for 16 hours using a 3.5 cm length (L) by 0.5 cm width (W) magnetic stirrer bar at 500 rpm speed. The mixture is filtered and the filtrate is then passed through a NUCLEOSIL C8 100A reverse phase column (available from Column Engineering Incorporated, a business having offices located in Ontario, California, U.S.A.) to separate the acrylic acid monomer. The acrylic acid monomer elutes at a certain time with a detection limit at about 10 ppm. The peak area of resulting elutes calculated from the chromatogram is then used to calculate the amount of residual acrylic acid monomer in the film. Initially, a calibration curve is generated by plotting the response area of pure acrylic acid elutes against its known amount (ppm). A linear curve with a correlation coefficient of greater than 0.996 is obtained.

### <u>16 Hr Extractable Test (%)</u>

The following test methods are used to calculate the 16-hour extractable levels for the superabsorbent polymer solution. The first test method is intended for use on carboxylic acid based superabsorbent materials. About 0.5 g of cure film obtained from the superabsorbent polymer solution is placed into a 250 ml conical flask containing 100 ml 0.9% NaCl solution. The mixture is stirred with a 3.5 cm L x 0.5 cm W magnetic stirrer bar at 500 rpm speed for 16 hours. The sample is then filtered using WHATMAN #3 filter paper (available from Whatman, Inc., a business having offices located in Florham Park, New Jersey, U.S.A.) and an aspirator attached to a water faucet that creates a vacuum in the filtration unit by sucking air with running water. The entire solution is filtered and special care is taken to ensure that no fluid is lost, and that no solid material passes through or around the filter paper. Approximately 50 g of the filtered solution is then taken into a 100 ml beaker. The pH of the solution is adjusted to 8.5 stepwise by using 1.0N NaOH and 0.1 N HCl. The resulting solution is then titrated to a pH of 3.9 using a BRINKMANN TITROPROCESSOR (available from Brinkmann Instruments, Inc., a business having offices located in Westbury, New York, U.S.A.). The results are calculated by weight basis, with an assumed sodium/hydrogen acrylate formula weight of 87.47. The formula weight is derived from that of 70% neutralized acrylic acid.

**Centrifuge Retention Capacity (CRC) Test**

As used herein, the Centrifuge Retention Capacity (CRC) is a measure of the Absorbent Capacity of the flexible superabsorbent binder polymer composition retained after being subjected to centrifugation under controlled conditions. The CRC can be measured by placing a sample of the material to be tested into a water-permeable bag that will contain the sample while allowing the test solution (0.9 percent NaCl solution) to be freely absorbed by the sample. A heat-sealable tea bag material (available from Dexter Nonwovens of Windsor Locks, Conn., U.S.A., as item #11697) works well for most applications. The bag is formed by folding a 12.7 by 7.6 cm (5-inch by 3-inch) sample of the bag material in half and heat sealing two of the open edges to form a 6.4 by 7.6 cm (2.5-inch by 3-inch) rectangular pouch. The heat seals should be about 6.4 mm (0.25 inch) inside the edge of the material. After the sample is placed in the pouch, the remaining open edge of the pouch is also heat-sealed. Empty bags are also made to be tested with the sample bags as controls. A sample size is chosen such that the teabag does not restrict the swelling of the material, generally with dimensions smaller than the sealed bag area (about 5 cm by 6.4 cm (2-inch by 2.5-inch)). Three sample bags are tested for each material.

The sealed bags are submerged in a pan of 0.9 percent NaCl solution. After wetting, the samples remain in the solution for 60 minutes, at which time they are removed from the solution and temporarily laid on a non-absorbent flat surface.

The wet bags are then placed into the basket of a suitable centrifuge capable of subjecting the samples to a g-force of 350. (A suitable centrifuge is a HERAEUS LABOFUGE 400, Heraeus Instruments, part number 75008157, available from Heraeus Infosystems GmbH, Hanau, Germany). The bags are centrifuged at a target of 1600 rpm, but within the range of 1500-1900 rpm, for 3 minutes (target g-force of 350). The bags are removed and weighed. The amount of fluid absorbed and retained by the material, taking into account the fluid retained by the bag material alone, is the Centrifuge Retention Capacity of the material, expressed as grams of fluid per gram of material.

**Viscosity After 16 Hours**

Viscosity of the flexible superabsorbent binder polymer solution is measured using a BROOKFIELD DVII+ PROGRAMMABLE viscometer (available from Brookfield Engineering, a business having offices located at Middleboro, Massachusetts, U.S.A.). Approximately 200-250 ml of binder composition is placed into a 709 g (25-ounce) plastic cup. The viscometer is generally zeroed initially with a desired Spindle. For binder composition, Spindle Number 3 is used. The viscosity is measured at 20 RPM and at temperature 22±1 °C.

**Percent Solids**

Approximately 20 ±0.5 g of flexible superabsorbent binder polymer composition is accurately weighed (W1) into a tared (W2) hexagonal plastic weighing dish. Approximate internal diameter (ID) of weighing dish is 12.7 cm/8.9 cm (5-inch/3.5-inch) (Top/Base). The polymer composition-containing dish is placed in a fuming hood at room temperature for 16-20 hours. The dish containing partially dried solid film is then placed into a laboratory oven preheated at 80°C for 30 minutes. The dish and its content are allowed to cool to room temperature. The dried dish with resulting solid film is then weighed together (W3). The percent solids is calculated using the following formula:

$$\%\ \text{Solids} = [(W3-W2)/(W1-W2)]\times 100$$

**Plate Stiffness Test**

Stiffness of the composites were measured using the "Zwick Flexibility" test. This test is a measure of stiffness of an article as it is deformed downward into a hole beneath the sample. For the test, the sample is modeled as an infinite plate with thickness t that resides on a flat surface where it is centered over a hole with radius R. A central force applied to the foam directly over the center of the hole deflects the foam down into the hole by a distance w when loaded in the center by a Force F. For a linear elastic material the deflection can be predicted by:

$$w = \frac{3F}{4\pi E t^3}(1-\nu)(3+\nu)R^2$$

where E is the effective linear elastic modulus, v is the Poisson's ratio, R is the radius of the hole, and t is the thickness of the foam, taken as the caliper in millimeters measured under a load of about 0.35 kPa, applied by a 7.6 cm diameter Plexiglass platen, with the thickness measured with a Sony U60A Digital Indicator. Taking Poisson's ratio as 0.1 (the solution is not highly sensitive to this parameter, so the inaccuracy due to the assumed value is likely to be minor), we can rewrite the previous equation for w to estimate the effective modulus as a function of the flexibility test results:

$$E \approx \frac{2R^2}{3t^3}\frac{F}{w}$$

The test results are carried out using an MTS Alliance RT/1 testing machine (MTS Systems Corp., Eden Prairie, Minnesota) with a 100 N load cell. As a an absorbent composite at least 6.25 cm by 6.25 cm square sits centered over a hole of radius 17 mm on a support plate, a blunt probe of 3.15 mm radius descends at a speed of 2.54 mm/min. When the probe tip descends to 1 mm below the plane of the support plate, the test is terminated. The maximum slope in grams of force/mm over any 0.5 mm span during the test is recorded (this maximum slope generally occurs at the end of the stroke). The load cell monitors the applied force and the position of the probe tip relative to the plane of the support plate is also monitored. The peak load is recorded, and E is estimated using the above equation.
The bending stiffness per unit width can then be calculated as:

$$S = \frac{Et^3}{12}$$

Those skilled in the art will recognize that the present invention is capable of many modifications and variations without departing from the scope thereof. Accordingly, the detailed description and examples set forth above are meant to be illustrative only and are not intended to limit, in any manner, the scope of the invention as set forth in the appended claims.

**EXAMPLES**

**Comparative Example 1**

A 2 L glass, jacketed reactor was initially purged with nitrogen. A circulating heater bath was equilibrated to 75 °C. In a 500 mL Erlenmeyer flask, the initiator system was prepared by dissolving benzoyl peroxide(BPO) (0.7105 g, 2.93 x $10^{-3}$ moles) in 250 mL of ethanol (MALLINCKRODT, completely denatured, available from Mallinckrodt Laboratory Chemicals, a division of Mallinckrodt Baker, Inc., a business having offices located in Phillipsburg, New Jersey, U.S.A.). In a 1 L pear-shaped flask, the monomer solution was prepared by mixing acrylic acid (56 mL, 0.817 moles), di(ethylene glycol) methyl ether methacrylate (62 mL, 0.336 moles) and 3-(trimethoxysilyl)propyl methacrylate (2.8 mL, 1.18 x $10^{-2}$) in 565 mL of ethanol (MALLINCKRODT, completely denatured). Nitrogen was then bubbled through the dissolved initiator system for 5 minutes, and the solution was transferred to the reactor. A positive pressure of nitrogen was maintained on the reactor. After the nitrogen was bubbled through the monomer solution for 5 minutes, a 30.5 cm (12-inch) needle connected to the inlet port of a MASTERFLEX peristaltic pump (available from Cole-Parmer Instrument Company, a business having offices located in Vernon Hills, Illinois, U.S.A.) was placed into the monomer solution. The outlet port of the MASTERFLEX pump was connected to a 30.5 cm (12-inch) needle which was inserted into the jacketed reactor. A positive pressure of nitrogen was maintained on the monomer flask.
The initiator system was heated to 75 °C with stirring by connecting the jacketed reactor to the circulating bath. When the internal temperature reached 60 °C, the monomer solution was added at a rate of about 3 g/min to the initiator system. The polymerization solution was stirred and heated at 75 °C for approximately 2 hours at which time a solution of azobisisobutyronitrile (AIBN) (0.1954 g, 1.19 x $10^{-3}$ moles) in 20 mL of ethanol was added. Stirring and heating at 75 °C was continued for an additional hour at which time a second solution of AIBN (0.1931 g, 1.18 x $10^{-3}$ moles) in 20 mL of ethanol was added to the polymerization solution. Stirring and heating at 75 °C was continued for an additional hour at which time a third solution of AIBN (0.1945 g, 1.18 x $10^{-3}$ moles) in 20 mL of ethanol was added to the polymerization solution. Stirring and heating was continued at 75 °C for a total reaction time of about 5 hours. The reactor was cooled to 35 °C in 30 min and the solution was drained into a 2 L plastic container.
To obtain about 70 mol% degree of neutralization for acrylic acid content of the resulting superabsorbent

polymer solution, an aqueous solution of sodium hydroxide (6.59 sodium hydroxide pellets dissolved in 40.8 g deionized (DI) water) was slowly added to 240 g of binder solution with constant stirring. The residual acrylic acid monomer was determined on films by using the Residual Monoethylenically Unsaturated Monomer Test method disclosed herein and was found to be 51,143. The CRC was also measured using the Centrifuge Retention Capacity Test method disclosed herein and was found to be 9.3g/g. The 16-hour extractables were also measured using the 16-Hr Extractables Test method disclosed herein and was found to be 15.3%.

**Comparative Example 2**

This comparative example was prepared using the following process. Solution No. 1 was prepared as follows: To 237 grams (3.289 moles) of acrylic acid was added 31.5 grams polyethylene glycol (mol. wt. = 200) and 52.6 grams of sodium hydroxide in 350 grams of water (40% neutralization) and 1.5 grams of ascorbic acid. This solution was cooled in an ice bath.

Solution No. 2 was prepared as follows: 31.5 grams polyethylene glycol (mol. wt. = 200) was diluted with 200 g water, then, with rapid stirring, 5 ml of 3-(trimethoxysilyl)propyl methacrylate(2.7 x $10^{-2}$ moles) was added to produce a hazy solution; then 3.15 g of 30% aqueous hydrogen peroxide was added to the solution.

Solution No. 3 was prepared by dissolving 39.5 grams (0.987moles) sodium hydroxide in 300 grams of water.

Solution No. 2 was added to Solution No. 1 in an ice bath while stirring with a magnetic stir bar. A thermocouple was used to monitor the temperature and observe the reaction exotherm. The polymerization reaction began after about 5 minutes of mixing. Once the exotherm reaction was detected, water was added gradually to keep the solution viscosity suitable for stirring. A total of 450 grams of water was added over 20 minutes. A maximum polymerization temperature of 85°C was observed about 8 minutes after mixing of the two monomer solutions. After about 20 minutes, Solution No. 3 was added with stirring to bring the neutralization to 70%, and was followed by additional water to reduce the polymer concentration to about 20%.

The resulting aqueous binder composition was cast into a film by pouring 22.6 grams of solution into a polystyrene weigh boat and allowing the water to evaporate overnight in a hood at room temperature, followed by drying in a Baxter Model No. DK-63 laboratory oven (available from Scientific Products, a division of Baxter Diagnostics, a business having offices located in McGraw Park, Illinois U.S.A.) at 50°C for 50 minutes. The resulting film weighed 5.95 grams, indicating a solution concentration of about 26%.

Comparative Example 2 resulted in the following properties: CRC of 11.9g/g, residual acrylic acid monomer of 5852 ppm and 16-hour extractables of 7.1%.

**Example 1** (not claimed)

A 1 L glass, jacketed reactor equipped with a thermometer and a mechanical stirrer system was initially purged with nitrogen. In a 500 mL Erlenmeyer flask, an initiator system was prepared by dissolving 2,2'-Azobis(2,4'-dimethylvaleronitrile)(V65B) (moles given in Table 2 below) in 125 mL of ethanol (MALLINCKRODT, completely denatured). In a 1 L beaker, the monomer solution was prepared by mixing acrylic acid (28 mL, 0.4085 moles), di(ethylene glycol) methyl ether methacrylate (31 mL, 0.168 moles) and 3-(trimethoxysilyl)propyl methacrylate (1.4 mL, 0.59 x $10^{-2}$) in 283 mL of ethanol (MALLINCKRODT, completely denatured). After nitrogen was bubbled through the dissolved initiator system as well as through the solution of monomer mixture for 5 minutes, both solutions were transferred to the reactor. A positive pressure of nitrogen was maintained on the reactor.

The reactor content was then heated to 55 °C under constant stirring at a modest speed. The reaction was continued for two hours. Then the reactor temperature was raised to 70 °C and a solution of t-amylperoxypivalate (TAPP) (moles given in Table 1) in 10 mL of ethanol was added. Stirring and heating at 70°C was continued for two additional hours. The total polymerization time was about 4 hours. The reactor was cooled to about 35 °C over 30 min and the solution was drained into a 2 L plastic container. To obtain a 70 mol% degree of neutralization for the acrylic acid content of the resulting superabsorbent polymer solution, an aqueous solution of sodium hydroxide (6.59 sodium hydroxide pellets dissolved in 40.8 g deionized (DI) water) was slowly added to 240 g of binder solution with constant stirring.

The results of Example 1 are shown in Table 2 below.

**Table 2.** Example 1

| Example | Initiator Package | Reaction Time (hrs) | Reaction Temperature °C | Degree of Neutralization (mol%) | CRC (g/g) | Residual Monomer (ppm) | 16 hour extr. (%) |
|---|---|---|---|---|---|---|---|
| 1 | V65B (0.1 mol/mol) | 2 | 55 | 70 | 12.9 | 428 | 28.2 |
| | TAPP (0.0043mol/mol) | 2 | 70 | | | | |

**Examples 2-6:** (not claimed)

Thermally decomposable initiators including sodium persulfate (NAPS), and 2,2'-azobis-2-amidinopropanedihydrchloride (ABAH) are included in addition to the redox initiator system in the amounts set forth in Table 3 below. Three solutions were then prepared separately.

Solution No. 1 was prepared as follows: To 237 grams (3.289 moles) of acrylic acid was added 31.5 grams polyethylene glycol (mol. wt. = 200) and 52.6 grams of sodium hydroxide in 350 grams of water (40% neutralization) and 1.5 grams of ascorbic acid. This solution was cooled in an ice bath.

Solution No. 2 was prepared as follows: 31.5 grams polyethylene glycol (mol. wt. = 200) was diluted with 200 g water, then, with rapid stirring 5 ml of 3-(trimethoxysilyl)propyl methacrylate(2.7 x $10^{-2}$ moles) was added to produce a hazy solution; then 3.15 g of 30% aqueous hydrogen peroxide was added to this solution.

Solution No. 3 was prepared by dissolving 39.5 grams (0.987moles) sodium hydroxide in 300 grams of water.

Solution No. 2 was added to Solution No. 1 in an ice bath while stirring with a magnetic stir bar. A thermocouple was used to monitor the temperature and observe the reaction exotherm. The polymerization reaction began after about 5 minutes of mixing. Once the exotherm reaction was detected, water was added gradually to keep the solution viscosity suitable for stirring. A total of 450 grams of water was added over 20 minutes. A maximum polymerization temperature of 75 °C was observed about 8 minutes after mixing of the two monomer solutions. After about 20 minutes, Solution No. 3 was added with stirring to bring the neutralization to 70%, followed by additional water to reduce the polymer concentration to about 20%.

The resulting aqueous polymer composition was cast into a film by pouring 22.6 grams of solution into a polystyrene weigh boat and allowing the water to evaporate overnight in a hood at room temperature, followed by drying in a Baxter Model No. DK-63 laboratory oven at 50 °C for 50 minutes. The resulting film weighed 5.95 grams, indicating a solution concentration of about 26%.

The results of Examples 2-6 are shown in Table 3 below.

**Table 3:** Examples 2-6

| **Example** | **$H_2O_2$ ppm** | **ASC ppm** | **NAPS ppm** | **ABAH ppm** | **CRC(g/g)** | **Residual acrylic acid monomer (ppm)** | **16 hr extract (%)** |
|---|---|---|---|---|---|---|---|
| 2 | 755 | 1199 | 600 | 600 | 11.9 | 557 | 22.1 |
| 3 | 755 | 1199 | 800 | 800 | 11.7 | 689 | 9.2 |
| 4 | 755 | 1199 | 400 | 400 | 10.9 | 697 | 10.2 |
| 5 | 755 | 1199 | 600 | none | 12.7 | 466 | 27.7 |
| 6 | 755 | 1199 | 800 | none | 11.2 | 235 | 29.1 |

The data in Table 3 shows that all of the thermal initiators decrease the residual acrylic acid monomer of the flexible superabsorbent binder polymer composition preparations to below 1,000 ppm.

**Examples 7-11**: (not claimed)

An aqueous solution of NaOH was prepared to obtain approximately 40% degree of neutralization for a desired amount of acrylic acid monomer by diluting 105.2 g of 50% aqueous NaOH in 1000 g of water. This solution was cooled

in an ice bath. An acrylic acid/PEG200 (polyethylene glycol, mol. wt.=200) was prepared by adding 237 g of glacial acrylic acid to 63.0 g PEG200 and mixed for about 5 minutes. This mixture was added to the NaOH solution and the resulting solution was cooled in an ice bath.

Initiator systems were prepared by dissolving 1.5 g ascorbic acid in 50 g deionized (DI) water, 1.0 g NAPS in 50 g DI water, and 2.7 g of 35% $H_2O_2$ diluted with 50 g DI water. The latent crosslinker solution was prepared just prior to initiation. With rapid stirring, 2.5 ml of 3-(trimethoxysilyl)propyl methacrylate (MEMO) was added to 100 g DI water producing a hazy solution. When the monomer solution reached 20 °C, the initiation sequence began. The monomer solution was removed from the ice bath prior to initiation. No further cooling was used in the rest of the polymerization process. The hydrogen peroxide solution, the NAPS solution, the crosslinker solution, and finally the ascorbic acid solution was added to the monomer solution. A thermometer was used to monitor the temperature and observe the reaction exotherm. The polymerization reaction began almost immediately once the initiator solutions were mixed under medium pace stirring with a mechanical power-stirrer. The reaction was allowed to proceed for 25 minutes. Then 79.0 g of 50% NaOH solution was added to post-neutralize the superabsorbent polymer solution to a final degree of neutralization of 70%.

The cross-linker MEMO was reduced by 50% in the above procedure. The amounts of sodium hydroxide and acrylic acid were varied to obtain various solid levels. The results are summarized in Table 4 below.

**Table 4:** Examples 7-11

| Sample ID | Acrylic Acid (% wt TMS) | % Solids film | CRC (g/g) | Residual acrylic acid monomer (ppm) | 16 hr Extr (%) | Viscosity After 16 hours (cPs) |
|---|---|---|---|---|---|---|
| 7 | 13.9 | 24.7 | 17.4 | 619 | 40.7 | 573 |
| 8 | 14.8 | 25.7 | 17.2 | 672 | 40.3 | 571 |
| 8 | 16.9 | 29.2 | 18.0 | 617 | 32.4 | 1700 |
| 9 | 16.9 | 30.6 | 13.8 | 283 | 23.6 | 7250 |
| 11 | 18.2 | 32.9 | 14.2 | 267 | 34.0 | 4420 |

**Examples 12-15:** (Invention)

In conjunction with Table 5 below for specific amounts of $H_2O_2$, Ascorbic Acid, and Hypophosphorus Acid, the following is the procedure for Examples 12-15.

Into a 1-gallon plastic bucket about 626.8 g water was added. To this water, 118.5 g of glacial acrylic acid was added. Then 52.8 g of 50% aqueous NaOH and 31.5 g PEG 200 were added. This solution mixture was cooled to 20-22 °C while sparging with $N_2$ gas. No cooling water or ice bath was used.

Initiator solutions were prepared as follows: (1) 1.04 g ascorbic acid was dissolved in 21.3 g water; (2) 0.5 g NAPS (sodium persulfate) was dissolved in 2.9 g water, (3) 1.93 g 35% $H_2O_2$ was weighed out.

A crosslinker solution was prepared just prior to initiation. With rapid stirring, 1.4 mL of 3-(trimethoxysilyl)propyl methacrylate (MEMO) was added to 21.3 g water producing a hazy solution. When the monomer solution reached 20-22 °C the initiation sequence began. To the monomer solution were added the hydrogen peroxide solution, the NAPS solution, 1.16 g of 50% w/w hypophosphorous acid (chain transfer agent), the crosslinker solution, and finally the ascorbic acid solution. The solution was stirred at medium pace with a mechanical stirrer. A thermocouple was used to monitor the temperature and observe the reaction exotherm. When the reaction reached it maximum temperature ($T_{max}$) (~50-55°C), 212.7 g water was added to the resulting polymer solution. The polymer solute on was allowed to cool while stirring was continued. No cooling water or ice bath was used.

When the polymer solution reached 25-27°C, the remaining 118.5 g glacial acrylic acid, 52.8 g 50% aq. NaOH, and 31.5 g PEG 200 were added to the solution. This solution mixture was allowed to cool to 25 - 27°C while sparging with $N_2$ gas. No cooling water or ice bath was used.

The remaining initiator solutions were prepared by dissolving 1.04 g ascorbic acid in 21.3 g water; dissolving 0.5 g NAPS (sodium persulfate) in 2.9 g water; weighing out 1.93 g 35% $H_2O_2$; and dissolving 1 g $Fe(SO_4)_3$ *$7H_2O$ in 100 g water. Then 1.0 g of the 1 % $FeSO_4$ solution was added to 5 g water.

The remaining crosslinker solution was prepared just prior to initiation. With rapid stirring, 1.4 mL of 3-(trimethoxysilyl)propyl methacrylate (MEMO) was added to 21.3 g water producing a hazy solution. In the second initiation step, the hydrogen peroxide solution, the NAPS solution, 1.16 g of 50% w/w hypophosphorous acid, the crosslinker solution,

the diluted iron sulfate solution, and finally the ascorbic acid solution were added to polymer/monomer solution mixture. The polymer/monomer solution mixture was stirred with a mechanical stirrer. A thermocouple was used to monitor the temperature and observe the reaction exotherm. The resulting polymer solution was allowed to cool after it reached its maximum temperature ($T_{max}$). No cooling water or ice bath was used. When the reaction solution reached 30 °C, 78.5 g of 50% NaOH solution was added to post-neutralize the polymer solution to a final degree of neutralization of 70%. The resulting polymer solution was stirred for approximately 5 minutes after addition of NaOH.

The results are summarized in Table 5 below.

**Table 5:** Examples 12-15

| | Step 1 | | | Step 2 | | | | Solution Properties | | Film Properties | | |
|---|---|---|---|---|---|---|---|---|---|---|---|---|
| Sample ID | $H_2O_2$ ppm | Asc. Acid ppm | HPA % wt AA | $H_2O_2$ ppm | Asc. Acid ppm | HPA % wt AA | Fe ppm | Visc. cps | Mol. WtMw g/mol $10^3$ | RM ppm | % Sol | CRC g/g |
| 9 | 1375 | 766 | - | 1836 | 1020 | - | 12.2 | 5980 | 372305 | 656 | 30.6 | 14 |
| 10 | 1375 | 766 | - | 1836 | 1020 | 2 | 12.2 | 1334 | 157296 | 21 | 33.4 | 16.6 |
| 11 | 1375 | 766 | - | 1836 | 1020 | 1 | 13.2 | 1943 | 230894 | 70 | 31.7 | 11.4 |
| 12 | 1375 | 766 | 0.5 | 1375 | 766 | 0.5 | 6.8 | 1277 | 174922 | 202 | 34.8 | 13.9 |
| TMS = Total Monomer Solution<br>Asc. A. = Ascorbic Acid<br>HPA = Hypo phosphorus Acid | | | | | | | | | | | | |

It can be seen from Table 5 that Samples 10 and 11 exhibit very low residual monomer levels.

**Example 16:** (not claimed)

The flexible superabsorbent binder polymer composition from Example 1 was applied to a spunbond fabric having a basis weight of 18 gsm (such as a diaper liner currently found in HUGGIES diapers, manufactured by Kimberly-Clark Corporation, having a place of business Neenah, Wisconsin, U.S.A.), and was treated with a surfactant solution mixture to provide wettability. The surfactant can be a 1:3 mixture of GLUCOPON 220 UP (available from Cognis Corporation, having a place of business in Cincinnati, Ohio, U.S.A) and AHCOVEL Base N-62 (available from Uniqema Inc., having a place of business in New Castle, Delaware, U.S.A.)., and the surfactant add-on level was 0.34% by weight. The solution of flexible superabsorbent binder polymer composition was applied by saturating a flexible sponge paint roller (available from Home Depot, having a place of business in Atlanta, Georgia, U.S.A.) with the flexible superabsorbent binder polymer solution. The spunbond fabric was then placed on a non-stick tray and rolled repeatedly with the saturated roller to saturate the fabric with the solution.

The coated fabric was placed on a grid-like tray made of polystyrene and dried in a Baxter Model No. DK-63 laboratory oven at 105 °C for 15 minutes. The basis weight of dried and crosslinked superabsorbent coating was 37 $g/m^2$.

The coated fabric was die cut into 4.44 mm diameter circles. The absorbent capacity of the coated fabric was test according the Centrifuge Retention Capacity test described above. The CRC of the coated fabric was measured to be 7.5 g/g.

**Example 17**: (Invention)

Initiator solutions were prepared as follows: (1) by dissolving 1.04 grams (g) of ascorbic acid in 21.3 g of water; (2) by dissolving 0.5 g of NAPS (sodium persulfate) in 2.9 g of water; and (3) by weighing out 1.93 g of 35% $H_2O_2$.

A crosslinker solution was prepared just prior to initiation. With rapid stirring, 1.4 mL of 3-(trimethoxysilyl)propyl methacrylate (MEMO) were added to 21.3 g of water producing a hazy solution.

A monomer solution was then prepared. While stirring at medium pace with a mechanical stirrer, approximately 626.8 g of water were added into a 1-gallon plastic bucket. To this water, 118.5 g of glacial acrylic acid were added. Then 52.8 g of 50% aqueous NaOH and 31.5 g of polyethylene glycol (PEG) with an average molecular weight of 200 were added and mixed. With continued mixing, this solution mixture was cooled to 20-22 °C while sparging with $N_2$ gas. No cooling water or ice bath was used. When the temperature of the monomer solution reached 20-22 °C, the initiation

sequence began. To the monomer solution were added the hydrogen peroxide solution, the NAPS solution, 1.16 g of 50% w/w hypophosphorous acid (chain transfer agent), the crosslinker solution, and finally the ascorbic acid solution. The solution was stirred at medium pace with a mechanical stirrer. A thermocouple was used to monitor the temperature and observe the reaction exotherm. When the reaction reached its maximum temperature (approximately 50-55 °C), 212.7 g of water were added to the resulting polymer solution. The polymer solution was allowed to cool while stirring was continued. No cooling water or ice bath was used.

When the polymer solution reached 25-27 °C, the remaining 118.5 g of glacial acrylic acid, 52.8 g of 50% aqueous NaOH, and 31.5 g of PEG 200 were added to the solution. This solution mixture was allowed to cool to 25-27 °C while sparging with $N_2$ gas. No cooling water or ice bath was used.

The remaining initiator solutions were prepared as follows: (1) by dissolving 1.04 g of ascorbic acid in 21.3 g of water; (2) by dissolving 0.5 g of NAPS (sodium persulfate) in 2.9 g of water; (3) by weighing out 1.93 g of 35% $H_2O_2$; and (4) by dissolving 1 g of $Fe(SO_4)_3$ *$7H_2O$ in 100 g of water. Then 1.0 g of the 1% $FeSO_4$ solution was added to 5 g of water.

The remaining crosslinker solution was prepared just prior to initiation. With rapid stirring, 1.4 mL of 3-(trimethoxysilyl)propyl methacrylate (MEMO) were added to 21.3 g of water producing a hazy solution.

In this second initiation step, while stirring at medium pace with a mechanical stirrer, the hydrogen peroxide solution, the NAPS solution, 1.16 g of 50% w/w hypophosphorous acid, the crosslinker solution, the diluted iron sulfate solution, and finally the ascorbic acid solution were added to the polymer/monomer solution mixture from above. A thermocouple was used to monitor the temperature and observe the reaction exotherm. The resulting polymer solution was allowed to cool after it reached its maximum temperature. No cooling water or ice bath was used. When the reaction solution reached 30 °C, 78.5 g of 50% NaOH solution were added to post-neutralize the superabsorbent polymer solution to a final degree of neutralization of 70%. The resulting polymer solution was stirred approximately 5 minutes after addition of NaOH.

A 21 gsm spunbond containing 2dTex (1.8 denier) polypropylene Spunbond fibers containing about 1% by weight $TiO_2$ and a wire weave bond pattern which was necked down 25%, and was treated with a surfactant solution mixture to provide wettability. The surfactant can be a 1:3 mixture of GLUCOPON 220 UP (available from Cognis Corporation, having a place of business in Cincinnati, Ohio, U.S.A) and AHCOVEL Base N-62 (available from Uniqema Inc., having a place of business in New Castle, Delaware, U.S.A.)., and the surfactant add-on level was 0.34% by weight.

The spunbond was immersed in the flexible superabsorbent binder polymer solution to thoroughly saturate the fabric. Excess fluid was squeezed out, and the saturated spunbond was dried for 4 minutes at 105 °C in a MATHIS through-air-dryer oven. After drying, the coated fabric had about a 41 gsm dry add-on of the dried flexible superabsorbent binder polymer composition.

The reduction in the stiffness of the absorbent layer was determined utilizing the Plate Stiffness Test described above. As shown in Table 6 below, there is a substantial reduction in stiffness when the absorbent layer is exposed to close-to-the-body conditions of 80% relative humidity compared to the "dried" condition that approximates the condition of the absorbent layer as it is assembled into the absorbent articles and packaged.

**Table 6 -** Plate Stiffness* of Absorbent layer as a Function of Sample Conditioning

| Sample Description | Plate Stiffness (N*mm) as a function of Sample Conditioning | | |
|---|---|---|---|
| 2 pli samples of: | Dried 100 °C (10 minutes) | 80% RH (10 minutes) | Percent Reduction at Body Conditions |
| Uncoated Spunbond 21 gsm | .23 | .25 | - |
| 41 GSM coating on 21 gsm spunbond, 20% PEG content | 2.4 | 0.63 | 81% |
| *Values in Table 6 are approximate. | | | |

**Claims**

**1.** An absorbent article comprising:

a backsheet and an absorbent layer adjacent to and in facing relationship with the backsheet,

wherein the absorbent layer comprises a flexible superabsorbent binder polymer composition;
wherein the flexible superabsorbent binder polymer composition comprises the reaction product of a monomer solution including:

at least 15% by mass monoethylenically unsaturated monomer selected from carboxylic acid, carboxylic acid salts, sulphonic acid, sulphonic acid salts,
phosphoric acid, or phosphoric acid salts;
an acrylate or methacrylate ester that contains an alkoxysilane functionality;
a plasticizer or a copolymerizable hydrophilic glycol containing an ester monomer;
an initiator system;
a chain transfer agent;
a transition metal salt; and
a neutralizing agent;
wherein the unsaturated monomer is neutralized to at least 25 mol%; and
wherein the flexible superabsorbent binder polymer composition has a weight average molecular weight of from 100,000 to 650,000 g/mole, a viscosity after 16 hours of less than 10,000 cps and a residual monoethylenically unsaturated monomer content of less than 1000 ppm.

**2.** The absorbent article of claim 1, wherein the chain transfer agent comprises hypophosphorous acid.

**3.** The absorbent article of claim 1 or 2, wherein, upon exposure to water, the alkoxysilane functionality forms a silanol functional group which condenses to form a crosslinked polymer.

**4.** The absorbent article of any of the preceding claims, wherein the monoethylenically unsaturated monomer comprises a carboxylic acid salt-containing monomer.

**5.** The absorbent article of any of the preceding claims, wherein the monoethylenically unsaturated monomer comprises between 20% and 99.9% by weight of the total monomer concentration to make the flexible superabsorbent absorbent binder composition.

**6.** The absorbent article of any of the preceding claims, wherein the acrylate or methacrylate ester comprises a monomer comprising a trialkoxysilane functional group.

**7.** The absorbent article of claim 6, wherein the monomer comprises at least one of methacryloxypropyl trimethoxy silane, methacryloxyethyl trimethoxy silane, methacryloxypropyl triethoxy silane, methacryloxypropyl tripropoxy silane, acryloxypropylmethyl dimethoxy silane, 3-acryloxypropyl trimethoxy silane, 3 methacryloxypropylmethyl diethoxy silane, 3-methacryloxypropylmethyl dimethoxy silane, 3-(trimethoxysilyl)propyl methacrylate , or 3-methacryloxypropyl tris(methoxyethoxy)silane.

**8.** The absorbent article of claim 6, wherein at least 0.1 % by weight of the flexible superabsorbent binder polymer composition comprises the monomer.

**9.** The absorbent article of any of the preceding claims, wherein the absorbent layer further comprises a substrate adjacent to and in facing relationship with the flexible superabsorbent binder polymer composition.

**10.** The absorbent article of any of the preceding claims, further comprising topsheet, wherein the topsheet is positioned such that the absorbent layer is positioned between the topsheet and the backsheet.

**11.** The absorbent article of any of claims 1-9, wherein the flexible superabsorbent binder polymer composition provides an outermost garment-facing surface.

**12.** The absorbent article of any of claims 1-9, wherein the flexible superabsorbent binder polymer composition provides an innermost bodyside surface.

**13.** The absorbent article of any of the preceding claims, wherein the plasticizer is polyethylene glycol.

**14.** The absorbent article of any of the preceding claims, wherein the neutralizing agent is sodium hydroxide.

15. The absorbent article of any of the preceding claims, wherein the acrylate or methacrylate ester that contains an alkoxysilane functionality is 3-(trimethoxysilyl)propyl methacrylate.

16. The absorbent article of any of the preceding claims, wherein the absorbent article is selected from personal care absorbent articles, health/medical absorbent articles or household/industrial absorbent articles.

**Patentansprüche**

1. Absorptionsfähiger Artikel, welcher umfasst:

   eine Rückschicht und eine absorptionsfähige Schicht, die angrenzend ist an die Rückschicht und der Rückschicht zugewandt ist,
   wobei die absorptionsfähige Schicht eine Zusammensetzung aus flexiblem superabsorptionsfähigem Bindemittelpolymer umfasst,
   wobei die Zusammensetzung aus flexiblem superabsorptionsfähigem Bindemittelpolymer das Reaktionsprodukt einer Monomerlösung umfasst, welche beinhaltet:

      mindestens 15 Masseprozent monoethylenisch nicht gesättigtes Monomer, welches ausgewählt ist aus Carbonsäure, Carbonsäuresalzen, Sulfonsäure, Sulfonsäuresalzen, Phosphorsäure oder Phosphorsäuresalzen;
      ein Acrylat oder Methacrylatester, der eine Alkoxysilan-Funktionalität enthält;
      einen Weichmacher oder ein copolymerisierbares hydrophiles Glykol, welches ein Estermonomer enthält;
      ein Initiatorsystem;
      ein Kettentransfermittel;
      ein Übergangsmetallsalz; und
      ein Neutralisierungsmittel;

   wobei das nicht gesättigte Monomer zu mindestens 25 Mol-% neutralisiert ist; und wobei die Zusammensetzung aus flexiblem superabsorptionsfähigem Bindemittelpolymer ein gewichtsgemitteltes Molekulargewicht von 100.000 bis 650.000 g/Mol, eine Viskosität nach 16 Stunden von weniger als 10.000 cps und einen Restgehalt monoethylenisch nicht gesättigter Monomere von weniger als 1.000 ppm aufweist.

2. Absorptionsfähiger Artikel gemäß Anspruch 1, wobei das Kettentransfermittel Hypophosphorige Säure umfasst.

3. Absorptionsfähiger Artikel gemäß Anspruch 1 oder 2, wobei die Alkoxysilan-Funktionalität bei Wasserexposition eine Silanol-funktionale Gruppe bildet, welche kondensiert, um ein vernetztes Polymer zu bilden.

4. Absorptionsfähiger Artikel gemäß einem der vorherigen Ansprüche, wobei das monoethylenisch nicht gesättigte Monomer ein Carbonsäuresalz-enthaltendes Monomer umfasst.

5. Absorptionsfähiger Artikel gemäß einem der vorherigen Ansprüche, wobei das monoethylenisch nicht gesättigte Monomer zwischen 20 Gew.-% und 99,9 Gew.-% der Gesamtmonomerkonzentration umfasst, um Zusammensetzung aus flexiblem superabsorptionsfähigem Bindemittelpolymer herzustellen.

6. Absorptionsfähiger Artikel gemäß einem der vorherigen Ansprüche, wobei das Acrylat oder der Methacrylatester ein Monomer umfasst, welches eine Trialkoxysilanfunktionale Gruppe umfasst.

7. Absorptionsfähiger Artikel gemäß Anspruch 6, wobei das Monomer mindestens eines umfasst aus Methacryloxypropyl-Trimethoxy-Silan, Methacryloxyethyl-Trimethoxy-Silan, Methacryloxypropyl-Triethoxy-Silan, Methacryloxypropyl-Tripropoxy-Silan, Acryloxypropylmethyl-Dimethoxy-Silan, 3-Acryloxypropyl-Trimethoxy-Silan, 3-Methacryloxypropylmethyl-Diethoxy-Silan, 3-Methacryloxypropylmethyl-Dimethoxy-Silan, 3-(Trimethoxysilyl)Propyl-Methacrylat oder 3-Methacryloxypropyl-Tris(Methoxyethoxy)Silan.

8. Absorptionsfähiger Artikel gemäß Anspruch 6, wobei mindestens 0,1 Gew.-% der Zusammensetzung aus flexiblem superabsorptionsfähigem Bindemittelpolymer das Monomer umfassen.

9. Absorptionsfähiger Artikel gemäß einem der vorherigen Ansprüche, wobei die absorptionsfähige Schicht des Weiteren ein Substrat umfasst, welches an die Zusammensetzung aus flexiblem superabsorptionsfähigem Bindemittelpolymer angrenzt und dieser zugewandt ist.

**10.** Absorptionsfähiger Artikel gemäß einem der vorherigen Ansprüche, welcher des Weiteren eine Oberseitenschicht umfasst, wobei die Oberseitenschicht derart angeordnet ist, dass die absorptionsfähige Schicht zwischen der Oberseitenschicht und der Rückschicht angeordnet ist.

**11.** Absorptionsfähiger Artikel gemäß einem der Ansprüche 1 bis 9, wobei die Zusammensetzung aus flexiblem superabsorptionsfähigem Bindemittelpolymer eine äußerste Bekleidungsoberfläche bereitstellt.

**12.** Absorptionsfähiger Artikel gemäß einem der Ansprüche 1 bis 9, wobei die Zusammensetzung aus flexiblem superabsorptionsfähigem Bindemittelpolymer eine innerste dem Körper zugewandte Oberfläche bereitstellt.

**13.** Absorptionsfähiger Artikel gemäß einem der vorherigen Ansprüche, wobei der Weichmacher Polyethylenglykol ist.

**14.** Absorptionsfähiger Artikel gemäß einem der vorherigen Ansprüche, wobei das Neutralisierungsmittel Natriumhydroxid ist.

**15.** Absorptionsfähiger Artikel gemäß einem der vorherigen Ansprüche, wobei das Acrylat oder der Methacrylatester, der eine Alkoxysilan-Funktinoalität enthält, 3-(Trimethoxysilyl)Propyl-Methacrylat ist.

**16.** Absorptionsfähiger Artikel gemäß einem der vorherigen Ansprüche, wobei der absorptionsfähige Artikel ausgewählt ist aus Körperpflege-Absorptionsartikeln, Gesundheits-/Medizinabsorptionsartikeln oder Haushalts-/Industrieabsorptionsartikeln.

## Revendications

**1.** Article absorbant comprenant :

une feuille postérieure et une couche absorbante adjacente à et faisant face à la feuille postérieure,
dans lequel la couche absorbante comprend une composition polymère liante, superabsorbante, flexible ;
dans lequel la composition polymère liante, superabsorbante, flexible comprend le produit réactionnel d'une solution de monomères contenant :

au moins 15 %mass d'un monomère à insaturation monoéthylénique sélectionné parmi l'acide carboxylique, les sels d'acide carboxylique, l'acide sulfonique, les sels d'acide sulfonique, l'acide phosphorique, ou les sels d'acide phosphorique ;
un ester acrylate ou méthacrylate qui contient une fonctionnalité alcoxysilane ;
un plastifiant ou un glycol hydrophile copolymérisable contenant un monomère ester ;
un système initiateur ;
un agent de transfert de chaîne ;
un sel de métal de transition ; et
un agent de neutralisation ;
dans lequel le monomère insaturé est neutralisé jusqu'à au moins 25 %mol ; et
dans lequel la composition polymère liante, superabsorbante, flexible a une masse moléculaire moyenne en poids de 100 000 à 650 000 g/mole, une viscosité après 16 heures inférieure à 10 000 cps et une teneur résiduelle en monomère à insaturation monoéthylénique inférieure à 1 000 ppm.

**2.** Article absorbant selon la revendication 1, dans lequel l'agent de transfert de chaîne comprend l'acide hypophosphoreux.

**3.** Article absorbant selon la revendication 1 ou 2, dans lequel, quand elle est exposée à l'eau, la fonctionnalité alcoxysilane forme un groupe fonctionnel silanol qui se condense pour former un polymère réticulé.

**4.** Article absorbant selon l'une quelconque des revendications précédentes, dans lequel le monomère à insaturation monoéthylénique comprend un monomère contenant un sel d'acide carboxylique.

5. Article absorbant selon l'une quelconque des revendications précédentes, dans lequel le monomère à insaturation monoéthylénique représente 20 % à 99,9 % en poids de la concentration totale en monomère pour former la composition polymère liante, superabsorbante, flexible.

6. Article absorbant selon l'une quelconque des revendications précédentes, dans lequel l'ester acrylate ou méthacrylate comprend un monomère contenant un groupe fonctionnel trialcoxysilane.

7. Article absorbant selon la revendication 6, dans lequel le monomère comprend au moins l'un du méthacryloxypropyltriméthoxysilane, du méthacryloxyéthyltriméthoxysilane, du méthacryloxypropyltriéthoxysilane, du méthacryloxyprvpyltripropoxysilane, de l'acryloxypropylméthyldiméthoxysilane, du 3-acryloxypropyltriméthoxysilane, du 3-méthacryloxypropylméthyldiéthoxysilane, du 3-méthacryloxypropylméthyldiméthoxysilane, du méthacrylate de 3-(triméthoxysilyl)propyle, ou du 3-méthacryloxypropyltris(méthoxyéthoxy)silane,

8. Article absorbant selon la revendication 6, dans lequel au moins 0,1 % en poids de la composition polymère liante, superabsorbante, flexible comprend le monomère.

9. Article absorbant selon l'une quelconque des revendications précédentes, dans lequel la couche absorbante comprend en outre un substrat adjacent à et faisant face à la composition polymère liante, superabsorbante, flexible.

10. Article absorbant selon l'une quelconque des revendications précédentes, comprenant en outre une feuille supérieure, la feuille supérieure étant positionnée de telle sorte que la couche absorbante soit positionnée entre la feuille supérieure et la feuille postérieure.

11. Article absorbant selon l'une quelconque des revendications 1 à 9, dans lequel la composition polymère liante, superabsorbante, flexible forme une surface située le plus à l'extérieur et faisant face à un vêtement.

12. Article absorbant selon l'une quelconque des revendications 1 à 9, dans lequel la composition polymère liante, superabsorbante, flexible forme une surface située le plus à l'intérieur et faisant face au corps.

13. Article absorbant selon l'une quelconque des revendications précédentes, dans lequel le plastifiant est un polyéthylène glycol.

14. Article absorbant selon l'une quelconque des revendications précédentes, dans lequel l'agent de neutralisation est l'hydroxyde de sodium.

15. Article absorbant selon l'une quelconque des revendications précédentes, dans lequel l'ester acrylate ou méthacrylate qui contient une fonctionnalité alcoxysilane est le méthacrylate de 3-(triméthoxysilyl)propyle.

16. Article absorbant selon l'une quelconque des revendications précédentes, l'article absorbant étant sélectionné parmi des articles absorbants d'hygiène personnelle, des articles absorbants de santé/médicaux, ou des articles absorbants à usage ménager/industriel.

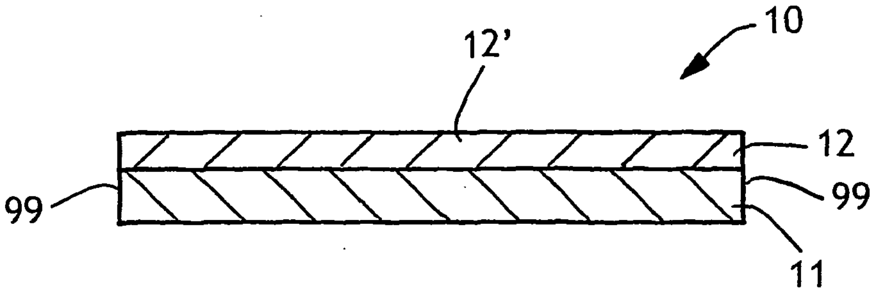


FIG. 1A

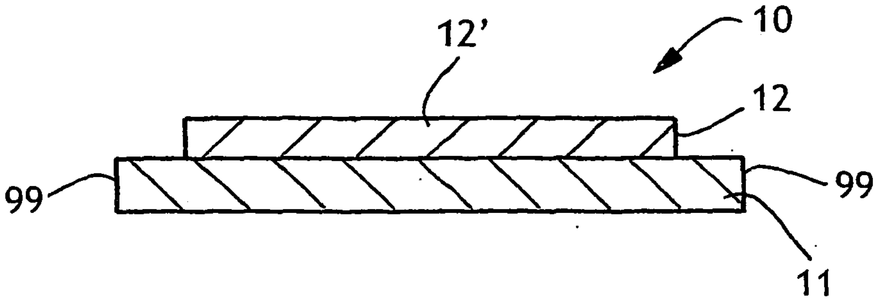


FIG. 1B

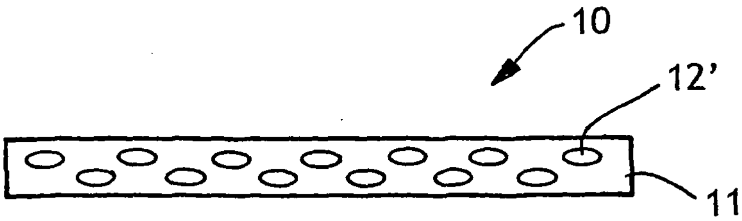


FIG. 1C

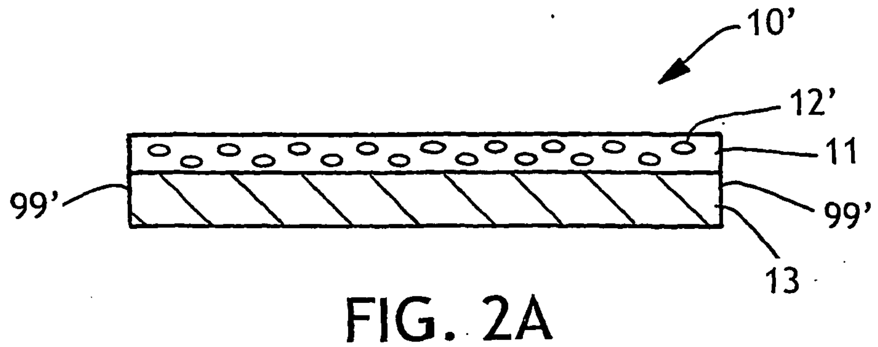


FIG. 2A

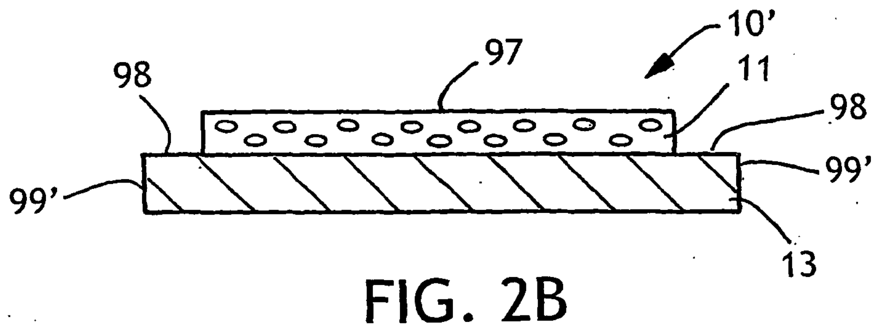


FIG. 2B

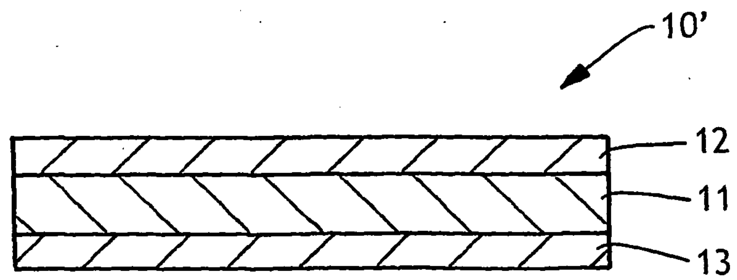


FIG. 2C

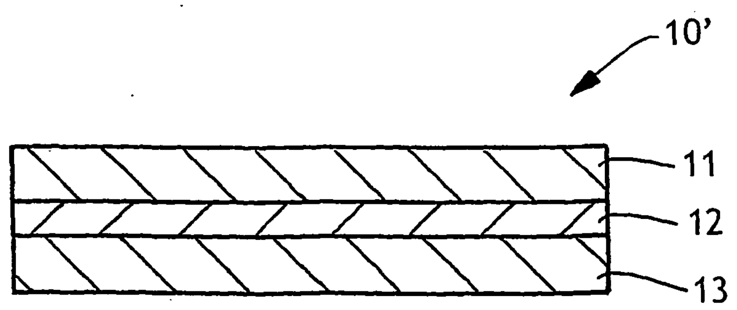


FIG. 2D

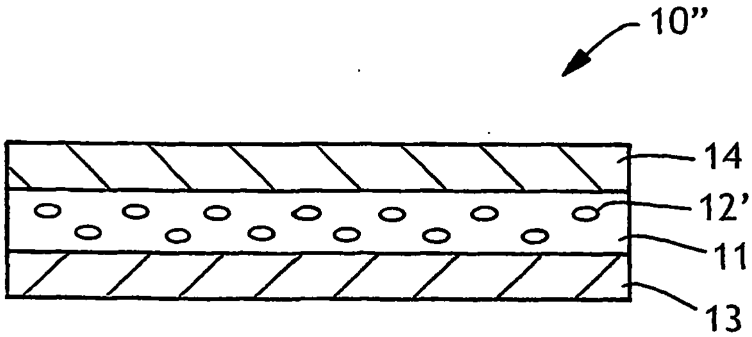


FIG. 3A

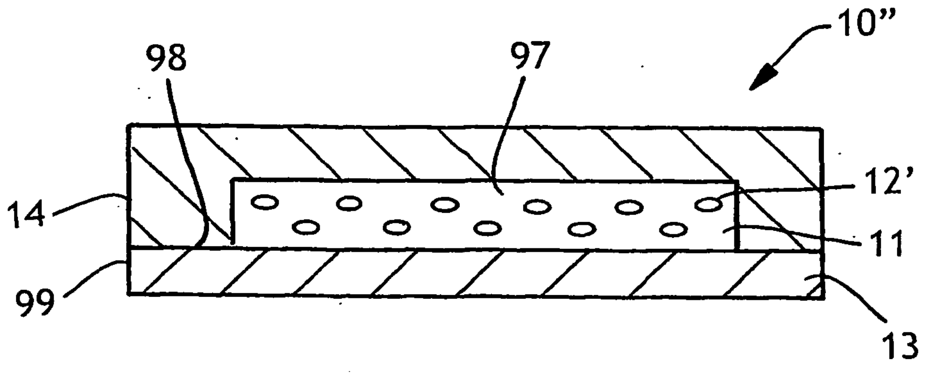


FIG. 3B

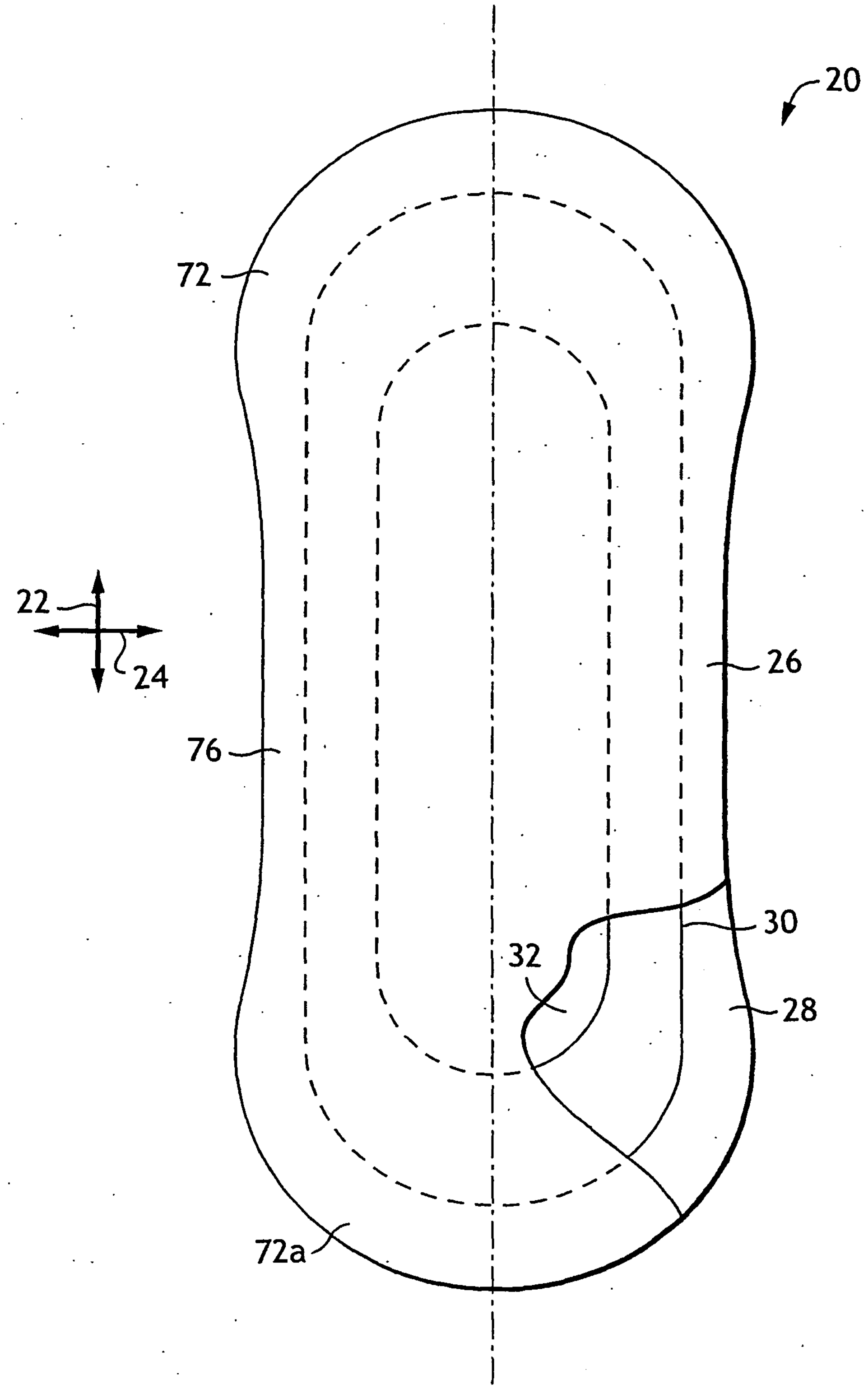


FIG. 4

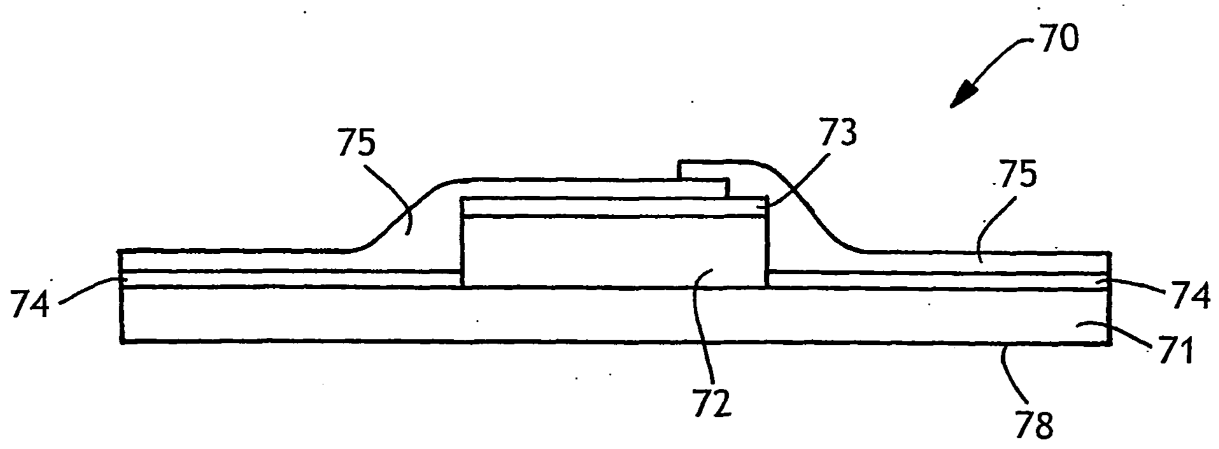


FIG. 5A

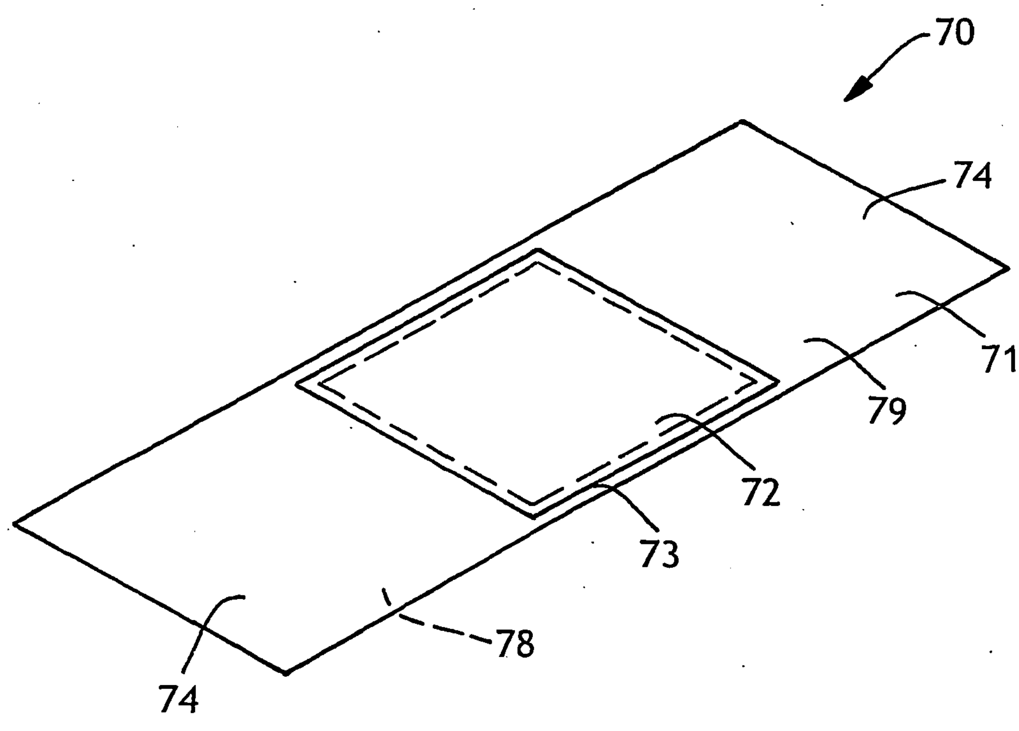


FIG. 5B

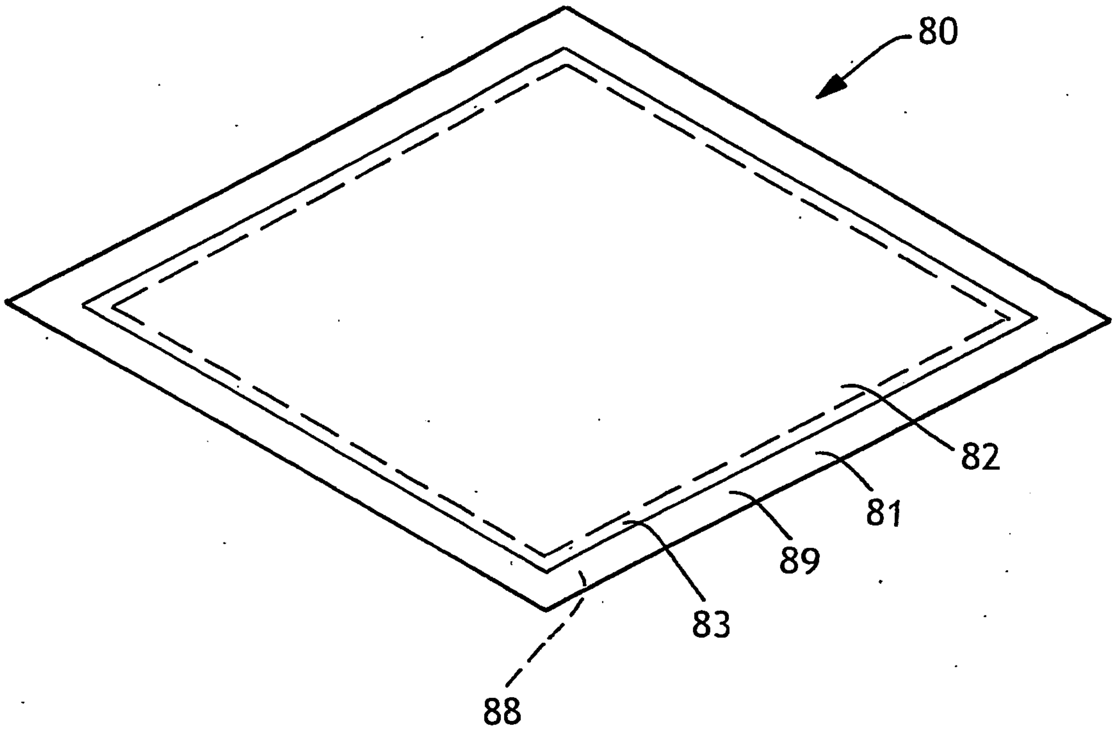


FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20040018365 A
- US 20040106721 A
- US 20040019166 A
- US 20040116014 A


### Non-patent literature cited in the description


- Surface and Colloid Science - Experimental Methods. Plenum Press, 1979, vol. II